# EUROPEAN PATENT SPECIFICATION

(11) **EP 4 119 308 B1**
(45) Date of publication and mention of the grant of the patent: **23.07.2025**
(21) Application number: 21768562.7
(22) Date of filing: 03.03.2021
(51) Int. Cl.: B25J 15/08, G06F 3/01, A61B 5/11, A61B 5/00, B25J 9/00, B25J 13/02, G06F 3/0346

(54) **INFORMATION PROCESSING DEVICE, INFORMATION PROCESSING METHOD, AND INFORMATION PROCESSING PROGRAM**
INFORMATIONSVERARBEITUNGSVORRICHTUNG, INFORMATIONSVERARBEITUNGSVERFAHREN UND INFORMATIONSVERARBEITUNGSPROGRAMM
DISPOSITIF DE TRAITEMENT D'INFORMATIONS, PROCÉDÉ DE TRAITEMENT D'INFORMATIONS ET PROGRAMME DE TRAITEMENT D'INFORMATIONS

(30) Priority: 11.03.2020 JP 2020041728
(43) Date of publication of application: 18.01.2023
(73) Proprietor: Sony Group Corporation, Tokyo 108-0075 (JP)
(72) Inventor: NISHIOKA, Hayato, Tokyo 141-0022 (JP); OKU, Takanori, Tokyo 141-0022 (JP); FURUYA, SHINICHI, Tokyo 141-0022 (JP)
(74) Representative: MFG Patentanwälte Meyer-Wildhagen Meggle-Freund Gerhard PartG mbB
(86) International application number: PCT/JP2021/008299
(87) International publication number: WO 2021/182264

(56) References cited:
- CN-A- 106 078 699
- JP-A- 2000 132 305
- JP-A- 2001 100 906
- JP-A- 2013 535 039
- JP-A- 2016 083 000
- US-A1- 2014 031 698
- US-A1- 2015 130 696
- US-A1- 2015 297 934
- US-A1- 2016 198 995
- US-B2- 9 743 860

## Description

### Field

The present invention relates to an information processing device, an information processing method, and an information processing program.

### Background

Technologies have been known to record and reproduce movements of fingers for the purpose of passing on excellent fine movements of the fingers of musical instrument players, traditional craft workers, cooks, surgeons, and the like to others (disciples, etc.) to support the proficiency of the others. For example, a technology is known to measure movement of a finger by mounting a small sensor including a triaxial acceleration sensor and a triaxial gyro sensor to each joint of the finger.

Patent Literature 2 discloses various embodiments relating to apparatuses and methods of using light transmission thought living tissue, such as a finger, to detect the flexure of a joint. Light is introduced into the tissue at one point, passes through the tissue, and exits the tissue at a second point where a sensor receives the light as it exits the tissue. Transmission of light through living tissue such as a finger can be affected by movement of the finger. As the finger flexes and, for example, the joints of the finger change angle, the characteristics of the light exiting the tissue, such as the intensity of the light, can change. These changes in characteristics can be used as an indirect means of determining the flexure of the joint.

Patent Literature 3 discloses a hand pressure reduction mechanical exoskeleton device which comprises a forearm fixing cylinder and a forearm rotating cylinder, wherein the front inner wall of the forearm fixing cylinder is provided with two annular grooves; the back of the forearm rotating cylinder is arranged in the forearm fixing cylinder; the outer wall of the forearm rotating cylinder is provided with two annular ribs which are rotatablely limited in the annular grooves; the front end of the forearm rotating cylinder is connected with a palm placement cylinder through a wrist universal joint; the left side or right side of the bottom of the palm arrangement cylinder is provided with a thumb protrusion port; and the top of the palm placement cylinder is respectively connected with a little finger exoskeleton assembly, a fourth finger exoskeleton assembly, a middle finger exoskeleton assembly and a forefinger exoskeleton assembly, which are positioned on the front side of the palm placement cylinder, through a single-drive multi-link mechanism side by side in a transmission mode. The hand pressure reduction mechanical exoskeleton device is specially designed for porters, sufficiently relieves the force of the hands of the porters, greatly increases the weight of the transported weights, and enhances the goods transportation efficiency. Besides, the hand pressure reduction mechanical exoskeleton device is also applicable to medical appliances, and is beneficial to better and more comprehensive rehabilitation treatment on the hands and wrists of the patient.

Patent Literature 4 discloses a wearable joint-action sensor detects actions of a joint that links a first body segment to a second body segment by using a proximity sensor worn on the first body segment to detect a separation between the proximity sensor and the first and/or second body segment. Actions of a hip joint can be detected by wearing the joint-action sensor on the waist, and actions of a finger joint can be detected by wearing the joint-action sensor on a finger. A waist-worn joint-action sensor incorporating a proximity sensor for detecting hip-joint actions and a triaxial accelerometer for detecting body actions can detect common physical activities, such as sitting, standing, dancing, push-ups, and sit-ups, that cannot be detected by a prior-art waist-worn or wrist-worn pedometer. A detected joint action can also be used for controlling a computing device, or for reminding a user of poor posture or of sitting for too long.

### Citation List

### Patent Literature

Patent Literature 1: JP 2014-54483 A
Patent Literature 2: US 9 743 860 B2
Patent Literature 3: CN 106 078 699 A
Patent Literature 4: US 2016/198995 A1

### Summary

### Technical Problem

However, in such conventional art as described above, it is not always possible to appropriately measure the movement of the finger. For example, fine movements of fingers, such as playing a musical instrument or performing a surgical operation, requires fine sensitivity of fingertips. However, for example, in the conventional art described above, measuring a finger joint angle requires to mount the sensor to the joint of the finger, and therefore, there is a possibility that the fine movement of the finger may be hindered due to the mounted sensor. Accordingly, in the conventional art described above, it is not always possible to appropriately measure the movement of the finger.

Therefore, the present disclosure proposes an information processing device, an information processing method, and an information processing program that are configured to appropriately measure the movement of a finger.

### Solution to Problem

According to a first aspect the present disclosure provides an information processing device in accordance with independent claim 1. According to a second aspect the present disclosure provides an information processing method in accordance with independent claim 14. According to a third aspect the present disclosure provides a computer program in accordance with independent claim 15. Further aspects are set forth in the dependent claims, the drawings, and the following description.

To solve the above problem, an information processing device comprising: a detection unit that detects angles of at least some of joints of a finger in a non-contact manner; and an estimation unit that estimates a posture of the finger based on the angles of the joints detected by the detection unit.

### Brief Description of Drawings

FIG. 1 is a diagram illustrating an example of information processing according to a first embodiment of the present disclosure.
FIG. 2 is a diagram illustrating a configuration example of an information processing system according to the embodiment.
FIG. 3 is a diagram illustrating a configuration example of an information processing device according to the embodiment.
FIG. 4 is an exemplary view of a finger sensor according to the embodiment.
FIG. 5 is a view of a unit for a finger portion of the finger sensor according to the embodiment where the entire unit is viewed from a lateral direction.
FIG. 6 is a view of the unit for the finger portion of the finger sensor according to the embodiment where a fingertip side portion of the unit is enlarged and viewed from the lateral direction.
FIG. 7 is a diagram illustrating an example of a method of calculating a finger joint angle according to the embodiment.
FIG. 8 is a diagram illustrating an example of a method of calculating an adduction/abduction angle of a finger according to the embodiment.
FIG. 9 is a perspective view of an exoskeletal robot according to the embodiment.
FIG. 10 is a side view of the exoskeletal robot according to the embodiment.
FIG. 11 is a diagram illustrating an exoskeleton unit corresponding to one finger, of the exoskeletal robot according to the embodiment.
FIG. 12 is a diagram illustrating an RCM mechanism of the exoskeleton unit corresponding to one finger, of the exoskeletal robot according to the embodiment.
FIG. 13 is a top view of the exoskeletal robot according to the embodiment.
FIG. 14 is a flowchart illustrating an information processing procedure according to the embodiment.
FIG. 15 is an exemplary view of a finger sensor according to a modification of the embodiment.
FIG. 16 is an exemplary view of a finger sensor according to a modification of the embodiment.
FIG. 17 is an exemplary view of a finger sensor according to a modification of the embodiment.
FIG. 18 is an exemplary view of a finger sensor according to a modification of the embodiment.
FIG. 19 is an exemplary view of a finger sensor according to a modification of the embodiment.
FIG. 20 is an exemplary view of a finger sensor according to a modification of the embodiment.
FIG. 21 is a perspective view of an exoskeletal robot according to a modification of the embodiment.
FIG. 22 is a side view of the exoskeletal robot according to the modification of the embodiment.
FIG. 23 is a top view of the exoskeletal robot according to the modification of the embodiment.
FIG. 24 is a bottom view of the exoskeletal robot according to the modification of the embodiment.
FIG. 25 is a diagram illustrating an example of information processing according to a second embodiment of the present disclosure.
FIG. 26 is a diagram illustrating a configuration example of an information processing device according to the embodiment.
FIG. 27 is a conceptual diagram illustrating a process of information processing according to the embodiment.
FIG. 28 is a flowchart illustrating an information processing procedure according to the embodiment.
FIG. 29 is a flowchart illustrating an information processing procedure according to the embodiment.
FIG. 30 is a flowchart illustrating an information processing procedure according to the embodiment.
FIG. 31 is an exemplary view of a screen display on a terminal device of a user with a result of the processing according to the embodiment displayed.
FIG. 32 is an exemplary view of a screen display on the terminal device of the user with a result of the processing according to the embodiment displayed.
FIG. 33 is a hardware configuration diagram illustrating an example of a computer implementing the functions of the information processing device.

### Description of Embodiments

The embodiment of the present disclosure will be described in detail below with reference to the drawings. Note that in the following embodiment, the same portions are denoted by the same reference numerals or symbols, and repetitive description thereof will be omitted.

Furthermore, the present disclosure will be described in the order of items shown below.
0. Introduction
1. First Embodiment
1.1. Overview of information processing system
1.2. Configuration example of Information processing device
1.3. Operation example of information processing system
1.4. Modifications
1.4.1. Finger sensor
1.4.2. Exoskeletal robot
2. Example
2.1. Overview of information processing system
2.2. Configuration example of information processing device
2.3. Operation example of information processing system
3. Others
4. Effects
5. Hardware configuration

### [0. Introduction]

Recording and reproducing excellent fine movements of the fingers of musical instrument players, traditional craft workers, cooks, and the like are very important to transmit skills of skilled persons to others (i.e., disciples). Likewise for supporting proficiency, it is very effective to record fast movements of the fingers and present the movements to the users, for intuitively passing on tacit knowledge.

However, high spatial resolution and high temporal resolution are required for recording fast and excellent fine movements of the fingers. However, when a device is directly mounted to each of the fingers for precise measurement, there is a problem that the fine movement is hindered.

Therefore, the present invention proposes a system using an exoskeletal robot and a measurement device that has reduced degree of inhibition of the fine movements although the device is directly mounted to a finger.

### [1. First Embodiment]

### [1.1. Overview of information processing system

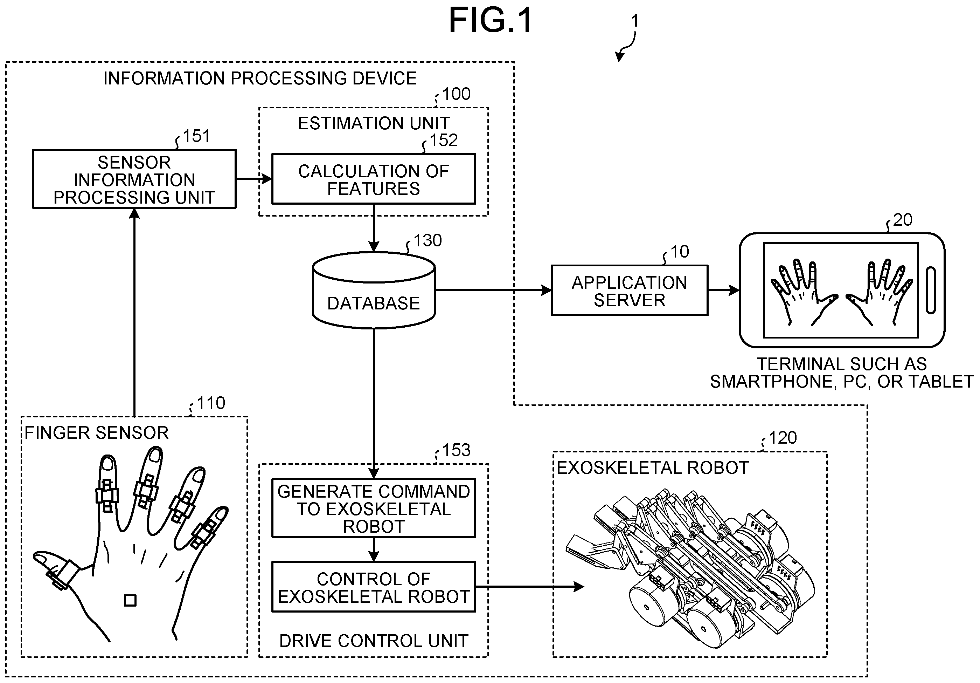

Hereinafter, an overview of information processing according to a first embodiment of the present disclosure will be described with reference to FIG. 1. FIG. 1 is a diagram illustrating an example of the information processing according to the first embodiment of the present disclosure. The information processing according to the first embodiment is performed by an information processing device 100 illustrated in FIG. 1.

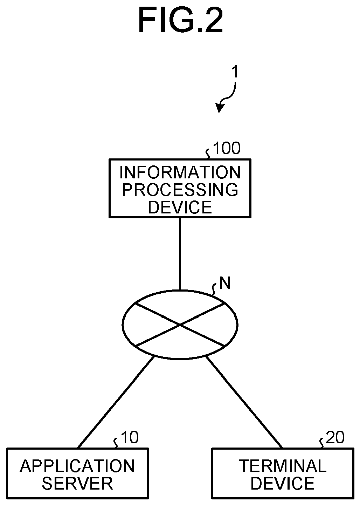

Prior to description of FIG. 1, a configuration of an information processing system according to the first embodiment of the present disclosure will be described with reference to FIG. 2. FIG. 2 is a diagram illustrating a configuration example of the information processing system according to the first embodiment of the present disclosure. As illustrated in FIG. 2, the information processing system 1 according to the first embodiment includes the information processing device 100, an application server 10, and a terminal device 20. The information processing device 100, the application server 10, and the terminal device 20 are communicably connected via a predetermined network N in a wired or wireless manner. Note that the information processing system 1 illustrated in FIG. 2 may include a plurality of the information processing devices 100, a plurality of the application servers 10, and a plurality of the terminal devices 20.

The application server 10 acquires finger feature information estimated by the information processing device 100, from the information processing device 100. The application server 10 having acquired the finger feature information generates content for presenting the finger feature information to the user. Subsequently, upon generating the content, the application server 10 delivers the generated content to the terminal device 20.

The terminal device 20 is an information processing device that is used by the user. For example, the terminal device 20 may be a device such as a smartphone, tablet terminal, notebook PC, desktop PC, a mobile phone, or a PDA. In the example illustrated in FIG. 1, the terminal device 20 is the smartphone.

The terminal device 20 presents information about the postures and positions of the fingers estimated by the information processing device 100 to the user. Specifically, the terminal device 20 transmits a content delivery request to the application server 10. Furthermore, the terminal device 20 receives the content from the application server 10. Upon receiving the content from the application server 10, the terminal device 20 outputs the content received from the application server 10.

The information processing device 100 is a server device that performs information processing according to the first embodiment. The information processing device 100 acquires sensor information about the intensity of reflected light from a finger to which light is emitted from a photoreflector of a finger sensor 110. Furthermore, the information processing device 100 acquires sensor information about an absolute angle of the middle phalanx of the finger relative to the ground surface, from a 9-axis posture sensor of the finger sensor 110, provided for the middle phalanx. Furthermore, the information processing device 100 acquires sensor information about an absolute angle of the back of the hand relative to the ground surface, from a 9-axis posture sensor of the finger sensor 110, provided for the back of the hand. Subsequently, the information processing device 100 estimates an angle of the DIP joint, PIP joint, or MP joint of the finger, as the finger feature information, on the basis of the acquired sensor information. Furthermore, the information processing device 100 presents the estimated finger feature information to the user. For example, the information processing device 100 transmits the estimated finger feature information to the application server 10, and presents the estimated finger feature information to the terminal device 20 of the user via the application server 10. Furthermore, the information processing device 100 presents the estimated finger feature information to the user by using an exoskeletal robot 120.

The overview of the information processing according to the first embodiment will be described below with reference to FIG. 1. In FIG. 1, the information processing device 100 includes a sensor information processing unit 151 that acquires the sensor information from the finger sensor 110. Furthermore, the information processing device 100 includes an estimation unit 152 that estimates the finger feature information on the basis of the sensor information acquired by the sensor information processing unit 151. For example, the estimation unit 152 estimates the finger feature information indicating such as an angle, average angular velocity, maximum angular velocity, and range of motion of each finger joint. Subsequently, upon estimating the finger feature information, the estimation unit 152 stores the estimated finger feature information in a storage unit 130.

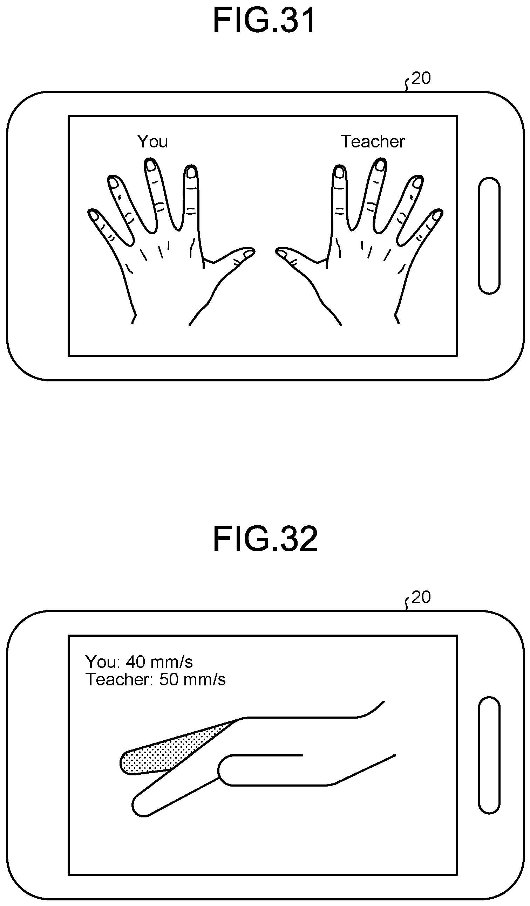

The application server 10 acquires the finger feature information from the storage unit 130. The application server 10 having acquired the finger feature information generates the content for presenting the finger feature information to the user, on the basis of the acquired information. For example, the application server 10 generates the content corresponding to images on a screen as illustrated in FIGS. 31 and 32 which are described later. In addition, the application server 10 generates the content to display the movements of the fingers at slow speed or fast speed. Furthermore, in presenting the movements of the fingers, the application server 10 generates the content to output sound according to the movements. For example, for the movements of the fingers playing piano, the application server 10 generates the content to output sound corresponding to keys struck by the fingers. Subsequently, upon generating the content, the application server 10 delivers the generated content to the terminal device 20.

The terminal device 20 receives the content from the application server 10. Upon receiving the content, the terminal device 20 outputs the received content. For example, the terminal device 20 displays the images on the screen as illustrated in FIGS. 31 and 32 which are described later. Furthermore, the terminal device 20 displays the movements of the fingers at slow speed or fast speed. Furthermore, when presenting the movements of the fingers, the terminal device 20 outputs sound according to the movements. For example, for the movements of the fingers playing piano, the terminal device 20 outputs sound corresponding to keys struck by the fingers.

Meanwhile, the information processing device 100 includes a drive control unit 153 that acquires the finger feature information from the storage unit 130. Subsequently, upon acquiring the finger feature information, the drive control unit 153 generates a command to the exoskeletal robot 120 on the basis of the acquired information. Then, upon generating the command to the exoskeletal robot 120, the drive control unit 153 controls the drive of the exoskeletal robot 120 on the basis of the generated command.

### [1.2. Configuration example of Information processing device]

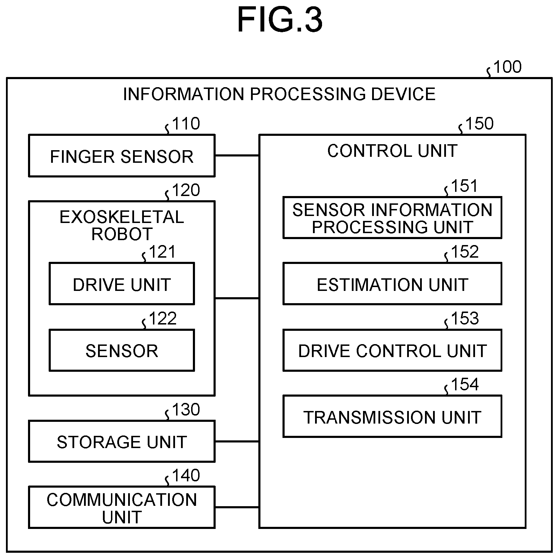

Next, a configuration of the information processing device according to the first embodiment of the present disclosure will be described with reference to FIG. 3. FIG. 3 is a diagram illustrating a configuration example of the information processing device according to the first embodiment of the present disclosure. As illustrated in FIG. 3, the information processing device 100 according to the first embodiment includes the finger sensor 110, the exoskeletal robot 120, the storage unit 130, a communication unit 140, and a control unit 150.

### (Finger sensor 110)

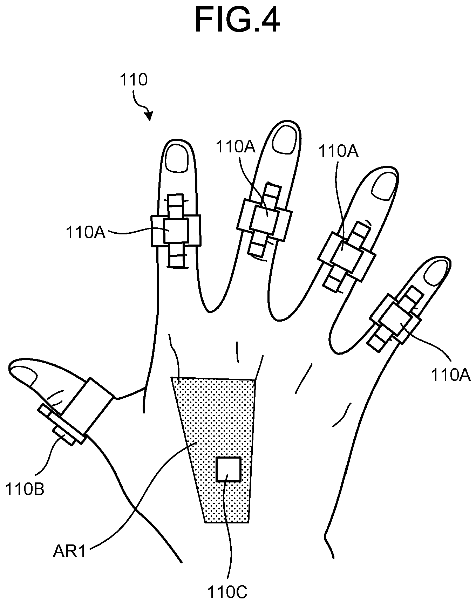

Next, a configuration of the finger sensor according to the first embodiment of the present disclosure will be described with reference to FIG. 4. FIG. 4 is an exemplary view of the finger sensor according to the first embodiment of the present disclosure. As illustrated in FIG. 4, the finger sensor 110 includes four first sensor units 110A for measuring postures of an index finger, middle finger, ring finger, and little finger, a second sensor unit 110B for measuring a posture of a thumb, and a 9-axis posture sensor 110C for measuring a reference posture as a reference of a finger joint angle.

The first sensor units 110A are mounted to the middle phalanxes of the index finger, middle finger, ring finger, and little finger. In the example illustrated in FIG. 4, each of the first sensor units 110A is mounted to an upper surface (dorsiflexion side) of the middle phalanx of each of the fingers by a ring-shaped band. As illustrated in FIG. 4, each first sensor unit 110A has a width that is designed to be smaller than the width of each finger. Furthermore, each first sensor unit 110A has a length that is designed to be larger than the length of the middle phalanx of each finger in a longitudinal direction by approximately 10%, and the photoreflector is provided at a portion that is designed to be longer by approximately 10% in the longitudinal direction. In addition, each first sensor unit 110A has a center portion to which a small 9-axis posture sensor having a thin square shape is attached.

The second sensor unit 110B is mounted to an upper surface (dorsiflexion side) of the proximal phalanx of the thumb. In the example illustrated in FIG. 4, a sensor for measuring the thumb is mounted on the upper surface (dorsal flexion side) of the proximal phalanx of the thumb by a ring-shaped band. The second sensor unit 110B has a rectangular shape as viewed from above. The second sensor unit 110B has a width that is designed to be smaller than the width of the thumb. Furthermore, the second sensor unit 110B has a length that is designed to be larger toward the MP joint than the length of the proximal phalanx of the thumb by approximately 10%, and the photoreflector is provided at a portion that is designed to be longer by approximately 10% toward the MP joint. Furthermore, the second sensor unit 110B has a center portion to which a small 9-axis posture sensor having a thin square shape is attached.

The 9-axis posture sensor 110C is attached to the back of the hand. In the example illustrated in FIG. 4, the 9-axis posture sensor 110C having a small thin square shape is attached to the back of the hand. Specifically, the 9-axis posture sensor 110C is mounted between the MP joints on the proximal phalanx bones of the index finger and the middle finger and the wrist, or mounted in an area therebetween (an area AR1 illustrated in FIG. 4) so as not to be affected by the bending of the palm of the hand or the movement of the thumb.

As described above, a second posture sensor (the 9-axis posture sensor 110C) is provided in the area between the MP joints on the proximal phalanx bones of the index finger and the middle finger and the wrist, on the back of the hand. This configuration makes it possible for the information processing device 100 to detect inclination of the back of the hand relative to the gravity direction so as not to be affected by the bending of the palm of the hand or the movement of the thumb.

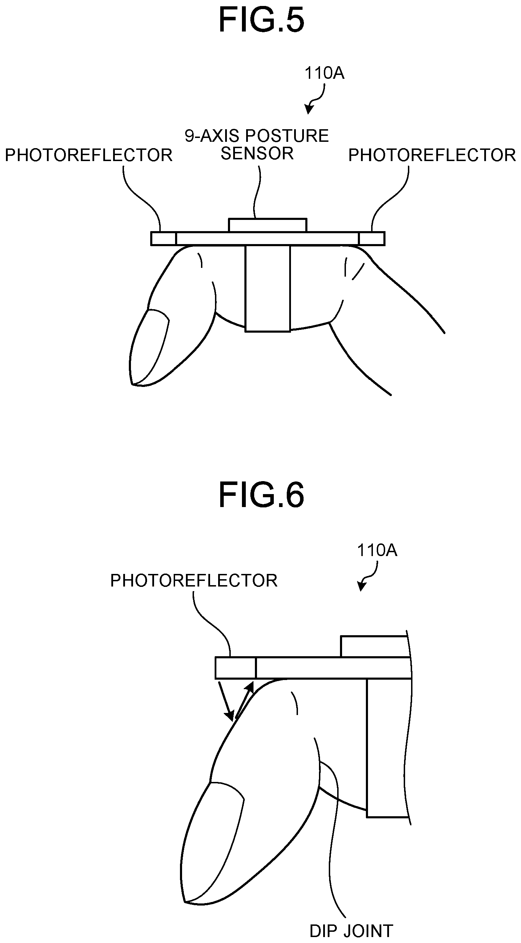

Next, a unit for a finger portion of the finger sensor according to the first embodiment of the present disclosure will be described with reference to FIG. 5. FIG. 5 is a view of the unit for a finger portion of the finger sensor according to the first embodiment of the present disclosure where the entire unit is viewed from a lateral direction. In the example illustrated in FIG. 5, the first sensor unit 110A includes, at the middle phalanx of each finger, one 9-axis posture sensor for measuring the absolute angle of the middle phalanx relative to the ground surface, and two photoreflectors for measuring a relative angle between the middle phalanx and a distal phalanx and a relative angle between the middle phalanx and a proximal phalanx. Note that in the following, the relative angle between the middle phalanx and the distal phalanx may be referred to as a DIP joint angle. In addition, the relative angle between the middle phalanx and the proximal phalanx may be referred to as a PIP joint angle.

Next, the unit for a finger portion of the finger sensor according to the first embodiment of the present disclosure will be further described with reference to FIG. 6. FIG. 6 is a view of the unit for a finger portion of the finger sensor according to the first embodiment of the present disclosure where a distal phalanx side portion of the unit is enlarged and viewed from the lateral direction. In the example illustrated in FIG. 6, the first sensor unit 110A emits infrared light from the photoreflector mounted on the extension of the upper surface of the middle phalanx and detects the intensity of light reflected and returned from the surface of the skin of the distal phalanx, measuring a distance between the sensor and the skin of the distal phalanx. Subsequently, the first sensor unit 110A calculates the DIP joint angle on the basis of the measured distance between the sensor and the skin of the distal phalanx. In this manner, the information processing device 100 is configured to measure the PIP joint angle and the DIP joint angle in a non-contact manner by using the photoreflectors.

As described above, a detection unit (the finger sensor 110) detects the angles of at least some of the joints of the finger in a non-contact manner. Therefore, the information processing device 100 performs detection in the non-contact manner, thus making it possible to detect the finger joint angles without hindering the sensitivity of the fingertips and the fine movements of the fingers. Therefore, the information processing device is configured to appropriately measure the movements of the fingers.

In addition, the detection unit includes a non-contact sensor (the photoreflector), detects the distance from the non-contact sensor to the finger that changes according to the angle of the finger joint, and thus detects the angle of the finger joint on the basis of the detected distance. In addition, the non-contact sensor includes a light emitting element that emits light and a light receiving element that detects reflected light of the light. The light emitting element emits light to the finger joint. The light receiving element detects reflected light of the light emitted to a finger phalanx. The detection unit detects the distance to the finger on the basis of the intensity of the reflected light detected by the light receiving element. In addition, the non-contact sensor is not limited to an optical sensor (light sensor) such as the photoreflector, and may be any sensor, as long as the non-contact sensor is a proximity sensor, such as an ultrasonic sensor or a capacitance sensor, that is configured to detect the distance from the sensor to the finger in the non-contact manner. Therefore, in the information processing device 100, detection of the distance from the non-contact sensor to the finger, instead of direct detection of the angle of each joint by mounting a sensor to each joint, makes it possible to indirectly (i.e., in a non-contact manner) detect the angle of the joint.

Furthermore, the non-contact sensor (photoreflector) is provided at the middle phalanx of at least one of the index finger, the middle finger, the ring finger, and the little finger. The light emitting element emits light to at least one of the distal phalanx and the proximal phalanx of the finger. The light receiving element detects the reflected light of the light emitted to at least one of the distal phalanx and the proximal phalanx of the finger. The detection unit calculates the relative angle of the distal phalanx of the finger to the middle phalanx of the finger, as the DIP joint angle of the finger, on the basis of the distance between the distal phalanx of the finger and the non-contact sensor calculated on the basis of the intensity of the reflected light. The estimation unit calculates the relative angle of the proximal phalanx of the finger to the middle phalanx of the finger, as the PIP joint angle of the finger, and estimates the posture of the finger on the basis of the DIP joint angle and the PIP joint angle of the finger. This configuration makes it possible for the information processing device 100 to detect the DIP joint angle and the PIP joint angle of the finger in the non-contact manner without mounting a sensor to the distal phalanx and the proximal phalanx of the finger.

In addition, the information processing device 100 requires only mounting the first sensor unit 110A and the second sensor unit 110B that have a small size to the middle phalanx and mounting the small 9-axis posture sensor 110C to the back of the hand. Therefore, it is possible to measure the PIP joint angle and the DIP joint angle with relatively low restriction, as compared with the conventional art.

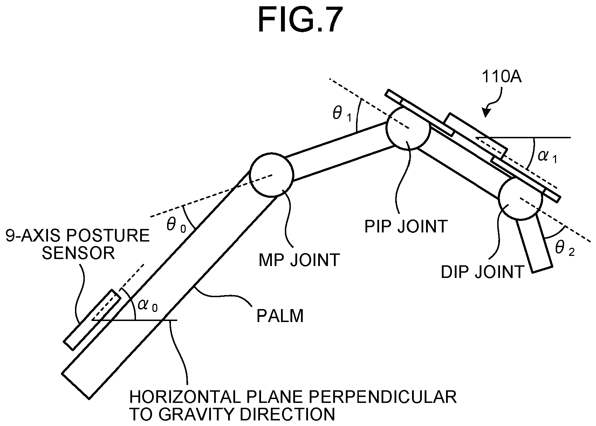

Next, a method of calculating a finger joint angle according to the first embodiment of the present disclosure will be described with reference to FIG. 7. FIG. 7 is a diagram illustrating an example of a method of calculating a finger joint angle according to the first embodiment of the present disclosure. FIG. 7 illustrates a model of one finger portion in which the palm and the proximal phalanx, middle phalanx, and distal phalanx of the finger are each regarded as a link, and the MP joint between the palm and the proximal phalanx, the PIP joint between the proximal phalanx and the middle phalanx, and the DIP joint between the middle phalanx and the distal phalanx are each regarded as a joint portion between the links.

An angle α₀ illustrated in FIG. 7 indicates an absolute angle of the palm of the hand relative to a horizontal plane measured by the 9-axis posture sensor attached to the back of the hand. An angle α₁ indicates an absolute angle of the middle phalanx relative to a horizontal plane measured by the 9-axis posture sensor mounted to the middle phalanx. In addition, an angle θ₀ indicates an MP joint angle that is a relative angle between the palm of the hand and the proximal phalanx. An angle θ₁ indicates the PIP joint angle that is the relative angle between the proximal phalanx and the middle phalanx. An angle θ₂ indicates the DIP joint angle that is the relative angle between the middle phalanx and the distal phalanx. At this time, the respective angles satisfy the relationship "α₀ - θ₀ - θ₁ = α₁". Therefore, the estimation unit 152 calculates the angle θ₀ of the MP joint to which the sensor is not mounted, on the basis of the angle α₀ and angle α₁ measured by the 9-axis posture sensors, the angle θ₁ calculated by the photoreflector, and the relationship of "α₀ - θ₀ - θ₁ = α₁". This configuration makes it possible for the information processing device 100 to calculate all the finger joint angles by using the 9-axis posture sensor attached to the back of the hand and the first sensor unit 110A attached to the finger.

As described above, the information processing device 100 further includes a first posture sensor (the 9-axis posture sensor provided at the middle phalanx of the finger) for detecting the inclination of the middle phalanx of the finger relative to the gravity direction. The estimation unit calculates the MP joint angle of the finger, on the basis of the PIP joint angle of the finger and the inclination of the middle phalanx of the finger relative to the gravity direction that is detected by the first posture sensor. The information processing device 100 further includes the second posture sensor (9-axis posture sensor 110C) for detecting the inclination of the back of the hand relative to the gravity direction. The estimation unit calculates, as the MP joint angle, a relative angle of the proximal phalanx of the finger to the back of the hand, on the basis of the inclination of the back of the hand relative to the gravity direction, detected by the second posture sensor. Therefore, this configuration makes it possible for the information processing device to calculate the MP joint angle in the non-contact manner without mounting a sensor to the MP joint.

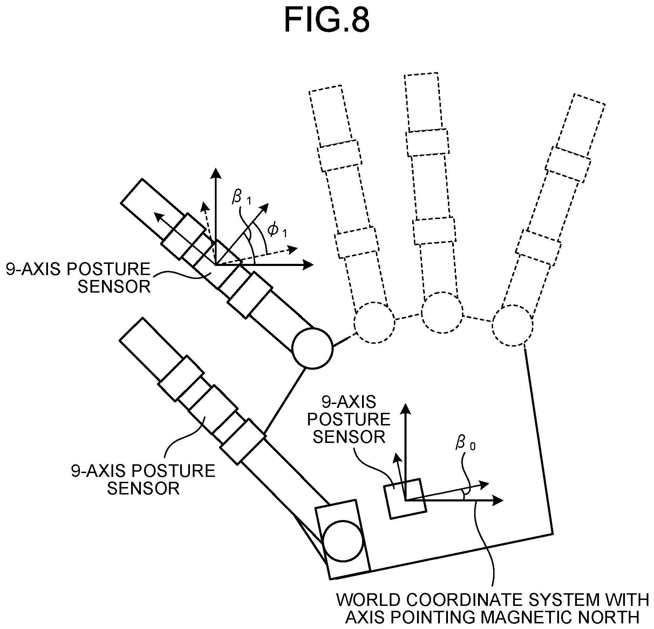

Next, a method of calculating an adduction/abduction angle of a finger according to the first embodiment of the present disclosure will be described with reference to FIG. 8. FIG. 8 is a diagram illustrating an example of the method of calculating an adduction/abduction angle of a finger according to the first embodiment of the present disclosure. As in FIG. 7, FIG. 8 illustrates a model of the entire one hand in which the finger phalanges are each regarded as the link and the joints of each finger are each regarded as the joint portion between the links.

An angle β₀ illustrated in FIG. 8 indicates an absolute angle of adduction or abduction of the palm of the hand in a world coordinate system with an axis pointing the magnetic north, and the absolute angle is measured by the 9-axis posture sensor attached to the back of the hand. In addition, an angle ϕ₁ indicates a relative angle of adduction or abduction of the finger in a reference coordinate system set to the back of the hand. In addition, an angle β₁ indicates an absolute angle of the adduction or abduction of the finger in the world coordinate system with an axis pointing the magnetic north, and the absolute angle is measured by the 9-axis posture sensor attached to the middle phalanx of the finger. At this time, the respective angles satisfy the relationship "ϕ₁ = β₁ - β₀". Therefore, the estimation unit 152 calculates, on the basis of the angle β₀ and angle β₁ measured by the 9-axis posture sensors, and the relationship of "ϕ₁ = β₁ - β₀", the relative angle ϕ₁ of the adduction or abduction of the finger in the reference coordinate system set to the back of the hand

As described above, the first posture sensor (9-axis posture sensor provided at the middle phalanx of the finger) detects the inclination of the adduction or abduction of the finger relative to the gravity direction. The second posture sensor (9-axis posture sensor attached to the back of the hand) detects the inclination of the adduction or abduction of the palm of the hand relative to the gravity direction. The estimation unit calculates information about the relative angle of the adduction or abduction of the finger to the back of the hand, on the basis of the inclination of the adduction or abduction of the finger relative to the gravity direction detected by the first posture sensor and the inclination of the adduction or abduction of the palm of the hand relative to the gravity direction detected by the second posture sensor. This configuration makes it possible for the information processing device 100 to estimate the angle of the adduction or abduction of the finger with relatively low constraint.

Next, a method of calculating the posture of the thumb will be described. The posture of the 9-axis posture sensor on the back of the hand, which has a reference attitude, is represented as a 3 × 3 rotation matrix M₀. Furthermore, the attitude of the 9-axis posture sensor attached to the distal phalanx of the thumb is represented as a 3 × 3 rotation matrix M₁. At this time, there is a 3 × 3 rotation matrix R that satisfies M₀ = RM₁. The rotation matrix R is a rotation matrix that represents a three-dimensional relative angle of the 9-axis posture sensor mounted to the distal phalanx of the thumb to the attitude of the 9-axis posture sensor on the back of the hand. In addition, the CM joint of the thumb has two degrees of freedom, and the MP joint of the thumb has one degree of freedom. The degrees of freedom of the thumb do not match each other in direction of the rotation axis. Therefore, the rotation angle of the thumb around each axis can be calculated from the three-dimensional rotation matrix R. Specifically, on the basis of the rotation matrix M₀ and rotation matrix M₁ measured by the 9-axis posture sensors and the relationship of "M₀ = RM₁", the estimation unit 152 calculates the rotation matrix R.

In addition, the first posture sensor described above is a 9-axis sensor that detects acceleration in three-axis directions, angular velocity in the three-axis directions, and orientation in the three-axis directions. In addition, the second posture sensor is a 9-axis sensor that detects acceleration in the three-axis directions, angular velocity in the three-axis directions, and orientation in the three-axis directions. This configuration makes it possible for the information processing device 100 to appropriately detect the inclination of the middle phalanx of the finger relative to the gravity direction and the inclination of the back of the hand relative to the gravity direction.

### (Exoskeletal robot 120)

The exoskeletal robot 120 reproduces the movements of the fingers detected by the finger sensor 110 according to the control of the drive control unit 153. Specifically, the exoskeletal robot 120 includes a drive unit 121 and a sensor 122.

### (Drive unit 121)

The drive unit 121 expresses the movements of the fingers by the exoskeletal robot 120. Therefore, the drive unit 121 includes an exoskeleton unit 120A and a motor 121A. For example, the drive unit 121 includes the exoskeleton unit 120A that corresponds to one finger and the motor 121A that drives the exoskeleton unit 120A. In the examples illustrated in FIG. 1 and FIGS. 9 to 13, the drive unit 121 includes four exoskeleton units 120A that correspond to the index finger, the middle finger, the ring finger, and the little finger, and four motors 121A that drive the four exoskeleton units 120A. For example, the drive unit 121 adduct or abduct the exoskeleton unit 120A around a rotation axis AX1 according to the control of the drive control unit 153. Furthermore, the drive unit 121 bends and stretches the exoskeleton units 120A around rotation axis of an RCM mechanism according to the control of the drive control unit 153.

### (Sensor 122)

The sensor 122 detects the posture and operation of the exoskeletal robot 120.a Specifically, the sensor 122 is implemented by a 9-axis posture sensor. For example, the sensor 122 detects an angle and an angular velocity of each joint that connects links of the exoskeleton unit 120A.

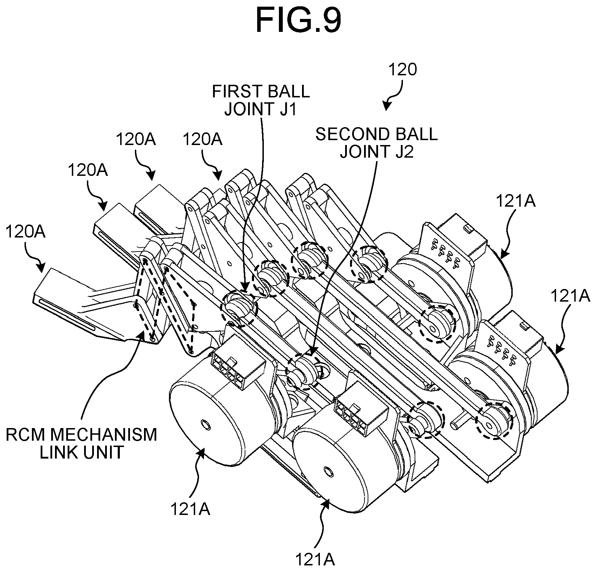

Next, the exoskeletal robot according to the first embodiment of the present disclosure will be described with reference to FIG. 9. FIG. 9 is a perspective view of the exoskeletal robot according to the first embodiment of the present disclosure. In the example illustrated in FIG. 9, the exoskeletal robot 120 includes the four exoskeleton units 120A corresponding to the index finger, the middle finger, the ring finger, and the little finger to reproduce the movements thereof, and the four motors 121A for driving the four exoskeleton units 120A corresponding to the respective fingers.

As illustrated in FIG. 9, in the exoskeletal robot 120, the links of adjacent fingers have lengths different from each other by the size of motor, and thus, the interference between the motors and a link mechanism can be eliminated.

In the conventional exoskeletal robots, the robots sometimes cannot be moved freely when wearers try to move the exoskeletal robots. This is mainly because that the frictional resistance of a drive transmission unit is large and the degrees of freedom of the fingers are restricted in the adduction or abduction. Therefore, the exoskeletal robot 120 according to the present invention solves these problems by using the motor 121A being powerful (specifically, a torque motor) with no speed reducer, and by using the link mechanism in which two ball joints (first ball joint J1 and second ball joint J2) are arranged at both ends of a link of the exoskeleton unit 120A corresponding to one finger and connected to an RCM mechanism link unit. In addition, the exoskeletal robot 120 according to the present invention hardly inhibits the movements of the fingers of the wearer unlike the conventional exoskeletal robots. Therefore, the movements of the fingers is also allowed to be recorded by the exoskeletal robot 120 by mounting a sensor on the exoskeletal robot 120.

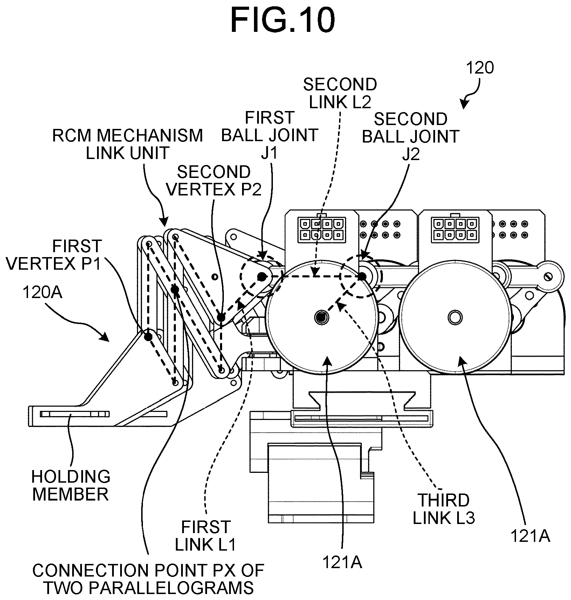

Next, the exoskeletal robot according to the first embodiment of the disclosure will be described with reference to FIG. 10. FIG. 10 is a side view of the exoskeletal robot according to the first embodiment of the present disclosure. As illustrated in FIG. 10, when the exoskeletal robot 120 is viewed from one lateral side, the exoskeleton unit 120A corresponding to one finger can be seen that is arranged on the back side of two motors 121A. Furthermore, the exoskeleton unit 120A corresponding to one finger includes the remote center of motion (RCM) mechanism link unit that has a double parallelogram structure using parallel links. Here, RCM stands for remote center of motion. In general, the RCM mechanism refers to a mechanism that has a rotation center at a position away from a rotation center of the motor to implement a pivot (fixed point) movement. For example, the RCM mechanism having the double parallelogram structure has a degree of freedom to rotate around the rotation center of the RCM mechanism in a plane including the double parallelogram. Furthermore, the exoskeleton unit 120A includes a first link L1 whose one end is fixed to a second vertex P2 positioned diagonally to a connection point PX of two parallelogram structures of the RCM mechanism link unit, a second link L2 whose one end is connected to another end of the first link L1 by the first ball joint J1, and a third link L3 whose one end is connected to another end of the second link L2 by the second ball joint J2. The first link L1, the second link L2, and the third link L3 constitutes a four-bar link structure. The exoskeleton unit 120A further includes the motor 121A, and the another of the third link L3 is fixed to a rotation shaft of the motor 121A.

The wearer who wears the exoskeletal robot 120 mounts the exoskeleton units 120A on the fingers so as to put a range from the MP joint to fingertip of each of the index finger, the middle finger, the ring finger, and the little finger along the RCM mechanism link unit. Specifically, the exoskeleton unit 120A includes a holding member that is fixed to a first vertex P1 positioned diagonally to the connection point PX of the two parallelogram structures of the RCM mechanism link unit and that holds the finger. Then, the index finger, the middle finger, the ring finger, and the little finger of the wearer of the exoskeletal robot 120 are held by the holding members of the exoskeleton units 120A corresponding to the respective fingers. The RCM mechanism link unit expands and contracts in accordance with bending and stretching movements of the fingers of the wearer.

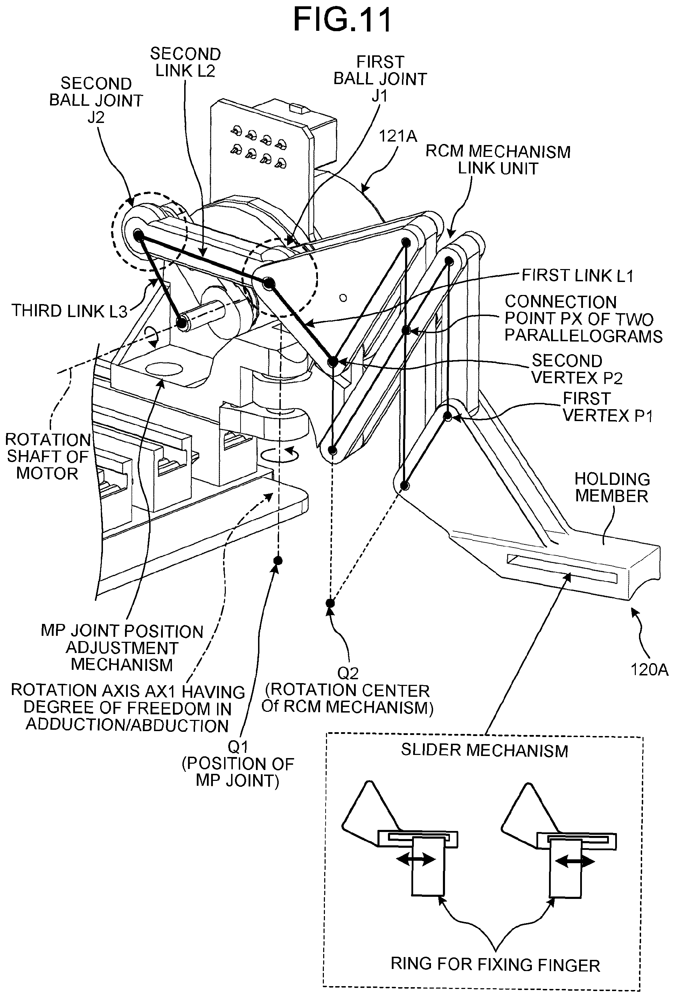

Next, the exoskeleton unit corresponding to one finger, of the exoskeletal robot according to the first embodiment of the present disclosure will be described with reference to FIG. 11. FIG. 11 is a diagram illustrating the exoskeleton unit corresponding to one finger, of the exoskeletal robot according to the first embodiment of the present disclosure. FIG. 11 is a view of the exoskeleton unit 120A corresponding to one finger, as viewed from a direction opposite to that of FIG. 10. In the example illustrated in FIG. 11, contrary to FIG. 10, the motor 121A can be seen that is arranged on the back side of the exoskeleton unit 120A corresponding to one finger. Here, the motor 121A is a direct drive or a motor having a small reduction ratio. Therefore, the exoskeletal robot 120 is configured so that the wearer can consciously move his/her fingers freely when power is off.

As illustrated in FIG. 11, the exoskeleton unit 120A includes an MP joint position adjustment mechanism. As described above, the exoskeleton unit 120A including the MP joint position adjustment mechanism makes it possible to adjust the position of the MP joint of each finger with orthogonal two degrees of freedom, in conformity with the shape of the hand of the wearer. Furthermore, in the exoskeleton unit 120A, the position of the MP joint of each finger, adjusted in conformity with the shape of the hand of the wearer, is configured to be fixed by a screw. As described above, each exoskeleton unit 120A has a width fit to the width of each finger, and the size of the exoskeleton units 120A is successfully fit to the size of the hand. In addition, the exoskeletal robot 120 is configured to adjust a rotation axis of a rotation mechanism that rotates the RCM mechanism according to the rotation of the adduction or abduction of the fingers so as to be positioned on the MP joint of each finger. This configuration makes it possible for the exoskeletal robot 120 to move the fingers with little restriction on the original movements of the fingers.

Furthermore, the exoskeleton unit 120A includes the holding member that is fixed to the first vertex P1 positioned diagonally to the connection point PX of the two parallelogram structures of the RCM mechanism link unit and that holds the finger. Furthermore, the exoskeleton unit 120A can further include a longitudinal slider mechanism as illustrated in the lower right of FIG. 11. For example, the exoskeleton unit 120A includes the slider mechanism that slidably holds a ring to fix the finger to the holding member. Thus, the exoskeleton unit 120A can cope with the fingers of the wearers having different lengths.

In addition, the exoskeleton unit 120A, as illustrated in FIG. 11, the exoskeleton unit 120A includes the rotation axis AX1 having the degree of freedom in the adduction or abduction of the finger, at a position immediately below the first ball joint J1. The exoskeleton unit 120A is configured to measure the adduction or abduction of the finger by a rotation sensor that is embedded to detect the rotation of the rotation axis AX1.

Furthermore, a point Q1 that is positioned at a lower end of the rotation axis AX1 illustrated in FIG. 11 indicates the position of the MP joint of the finger of the wearer. Specifically, in the example of the index finger as the one finger, the wearer who wears the exoskeletal robot 120 mounts the exoskeleton unit 120A so as to put the MP joint of the index finger at a position indicated by a black circle at the lower end of the rotation axis AX1 illustrated in FIG. 11. In addition, the wearer mounts the exoskeleton unit 120A on the index finger so as to put the range from the MP joint to fingertip of the index finger along the RCM mechanism link unit. The RCM mechanism link unit expands and contracts in accordance with the bending and stretching movements of the index finger of the wearer.

Furthermore, the exoskeleton unit 120A includes the RCM mechanism link unit having the double parallelogram structure using the parallel links. The RCM mechanism link unit includes the RCM mechanism. For example, the RCM mechanism link unit illustrated in FIG. 11 has a structure having connected links formed by combining the parallel links to form two bilaterally symmetrical parallelograms (also referred to as double parallelograms). Furthermore, the exoskeleton unit 120A includes the first link L1 whose one end is fixed to the second vertex P2 positioned diagonally to the connection point PX of two parallelogram structures of the RCM mechanism link unit, the second link L2 whose one end is connected to the other end of the first link L1 by the first ball joint J1, and the third link L3 whose one end is connected to the other end of the second link L2 by the second ball joint J2. The first link L1, the second link L2, and the third link L3 constitutes the four-bar link structure. The exoskeleton unit 120A further includes the motor 121A, and the other end of the third link L3 is fixed to the rotation shaft of the motor 121A.

A point Q2 below the RCM mechanism link unit illustrated in FIG. 11 indicates a position of the rotation center of the RCM mechanism of the RCM mechanism link unit. Furthermore, the position Q2 of the rotation center of the RCM mechanism is positioned on a symmetry axis of the double parallelograms. Furthermore, the position Q2 of the rotation center of the RCM mechanism is positioned between the MP joint and the PIP joint of the index finger of the wearer. As described above, the exoskeleton unit 120A includes the rotation axis of the RCM mechanism and the rotation axis of the rotation mechanism, within a predetermined range from the position of the MP joint of the finger. The rotation axis of the rotation mechanism rotates the RCM mechanism according to the rotation of the adduction or abduction of the finger.

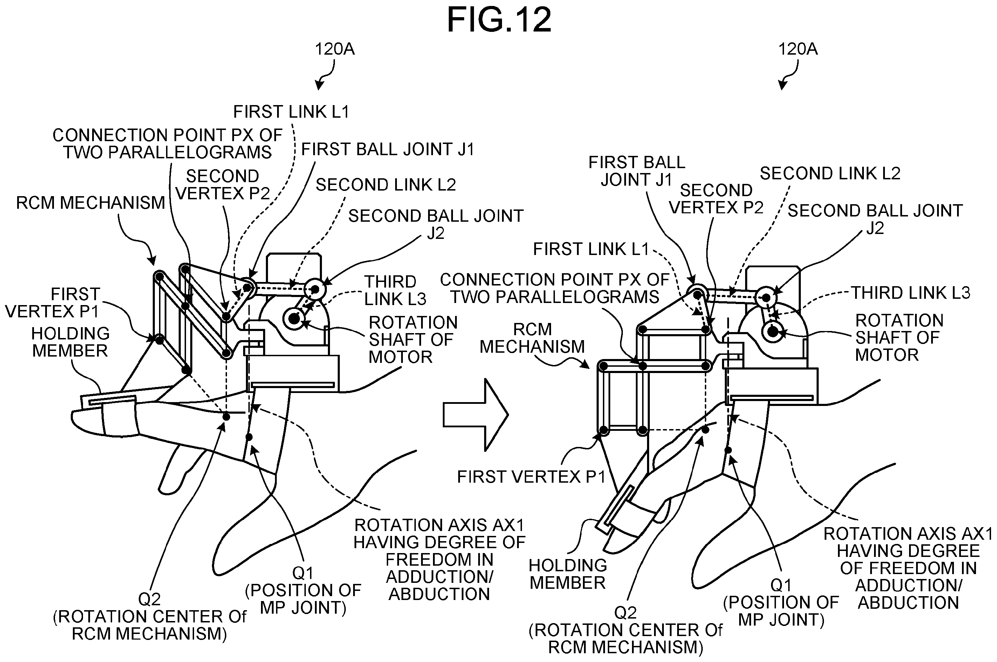

Next, the RCM mechanism of the exoskeleton unit corresponding to one finger, of the exoskeletal robot according to the first embodiment of the present disclosure will be described with reference to FIG. 12. FIG. 12 is a diagram illustrating the RCM mechanism of the exoskeleton unit 120A corresponding to one finger, of the exoskeletal robot 120 according to the first embodiment of the present disclosure. The left side of FIG. 12 illustrates a state where the finger is stretched. The right side of FIG. 12 illustrates a state where the finger is bent.

As illustrated in FIG. 12, the exoskeleton unit 120A includes the four-bar link having two ball bearings that receives the two ball joints J1 and J2. Specifically, the exoskeleton unit 120A includes the first link L1 whose one end is fixed to the second vertex P2 positioned diagonally to the connection point PX of two parallelogram structures of the RCM mechanism link unit, the second link L2 whose one end is connected to the other end of the first link L1 by the first ball joint J1, and the third link L3 whose one end is connected to the other end of the second link L2 by the second ball joint J2. The first link L1, the second link L2, and the third link L3 constitutes the four-bar link structure. The exoskeleton unit 120A further includes the motor 121A, and the other end of the third link L3 is fixed to the rotation shaft of the motor 121A. In addition, the exoskeleton unit 120A includes the rotation axis AX1 having the degree of freedom in the adduction or abduction of the finger, at a position immediately below the ball joint J2. Furthermore, the point Q1 at the lower end of the rotation axis AX1 corresponds to the position of the MP joint of the finger of the wearer. Therefore, in the exoskeletal robot 120, the frictional resistance of the drive transmission unit can be reduced and the degrees of freedom of the fingers can be improved in the adduction or abduction, and thus, it is possible to reproduce smoother and more natural movements of the fingers for the wearer of the exoskeletal robot 120.

Furthermore, the position Q2 of the rotation center of the RCM mechanism is positioned between the MP joint and the PIP joint of the finger of the wearer. In other words, the position Q2 of the rotation center of the RCM mechanism is positioned within a predetermined range from the MP joint of the finger of the wearer. As described above, the exoskeleton unit 120A includes the rotation axis of the RCM mechanism and the rotation axis of the rotation mechanism, within a predetermined range from the position of the MP joint of the finger. The rotation axis of the rotation mechanism rotates the RCM mechanism according to the rotation of the adduction or abduction of the finger. Here, in the exoskeleton unit 120A, as viewed from the wrist side of the wearer, the rotation axis AX1 having the degree of freedom in the adduction or abduction of an outer finger and the RCM mechanism are arranged in this order, in the degree of freedom of the device. Generally, the adduction or abduction of the finger is performed so that the finger rotates around the rotation axis AX1 at the MP joint. In addition, bending and stretching of the finger is performed so that the finger rotates around the rotation axis of the RCM mechanism at the MP joint, PIP joint, and DIP joint. Furthermore, the natural movements of the fingers can be considered as combination of the movements of adduction or abduction of the fingers and the movements of bending and stretching of the fingers. Therefore, when viewed from the wrist side, it can be considered that the natural movements of the fingers are performed in the order of the movements of the adduction or abduction of the fingers each rotating around the rotation axis AX1 and the movements of bending and stretching the fingers each rotating around the rotation axis of the RCM mechanism. The consideration makes it possible for the exoskeleton unit 120A having arrangement of the rotation axis AX1 having the degree of freedom in the adduction or abduction of an outer finger and the RCM mechanism in this order, as viewed from the wrist side of the wearer, to guide the finger as in the original movements of the finger. In other words, it is possible for the exoskeleton unit 120A to guide the movement of the adduction or abduction of the finger based on the MP joint of the finger and the movements of bending and stretching the finger based on the MP joint, the PIP joint, and the DIP joint, in the same manner as the original movements of the finger.

As described above, the information processing device 100 further includes the exoskeletal robot 120, and the exoskeletal robot 120 includes the RCM mechanisms each of which has the double parallelogram structure using the parallel links, the rotation mechanisms each of which rotates the RCM mechanism according to the rotation of the adduction or abduction of the finger, the holding members each of which is fixed to the first vertex P1 diagonally positioned to the connection point PX of the two parallelogram structures of the RCM mechanism to hold the finger, the first links L1 each of which has one end fixed to the second vertex P2 diagonally positioned to the connection point PX, the second links L2 each of which has one end connected to the other end of the first link L1 by the first ball joint J1, and the third links L3 each of which has one end connected to the other end of the second link L2 by the second ball joint J2, in which the first link L1, the second link L2, and the third link L3 constitute the four-bar link structure. Furthermore, the exoskeletal robot 120 further includes the motors 121A, and the other end of each third link L3 is fixed to the rotation shaft of each of the motors 121A. In addition, the rotation axis of the rotation mechanism and the rotation axis of the RCM mechanism are arranged in the order of the rotation axis of the rotation mechanism and the rotation axis of the RCM mechanism, from near the wrist of the wearer of the exoskeletal robot 120. In addition, the rotation axis of the rotation mechanism is positioned at the MP joint of the finger of the wearer of the exoskeletal robot, and the rotation axis of the RCM mechanism is positioned within the predetermined range from the MP joint of the finger, in a direction viewing the PIP joint from the MP joint of the finger of the wearer of the exoskeletal robot. This configuration makes it possible for the information processing device 100 having arrangement of the rotation axis having the degree of freedom in the adduction or abduction of the finger and the rotation axis of the RCM mechanism in this order, as viewed from the wrist side, to guide the finger as in the original movements of the finger. In other words, it is possible for the information processing device to guide the movement of the adduction or abduction of the finger based on the MP joint of the finger and the movements of bending and stretching the finger based on the MP joint, the PIP joint, and the DIP joint, in the same manner as the original movements of the finger.

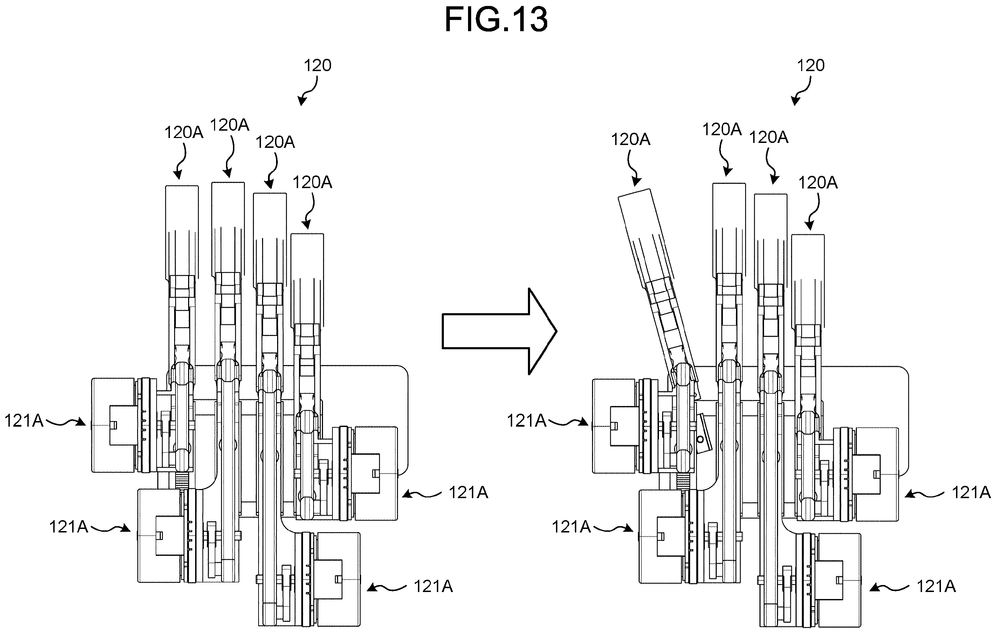

Next, the exoskeletal robot according to the first embodiment of the present disclosure will be described with reference to FIG. 13. FIG. 13 is a top view of the exoskeletal robot according to the first embodiment of the present disclosure. The left side of FIG. 13 is a top view of FIG. 9. The right side of FIG. 13 illustrates a state in which a screw of an MP joint position adjustment mechanism is loosened in the exoskeleton unit 120A corresponding to the index finger of the right hand and in which the exoskeleton unit 120A corresponding to the index finger of the right hand is moved.

### (Storage unit 130)

The storage unit 130 is implemented by, for example, a semiconductor memory device such as a random access memory (RAM) or flash memory, or a storage device such as a hard disk or optical disk. The storage unit 130 stores the sensor information acquired from the finger sensor 110. Furthermore, the storage unit 130 stores the finger feature information calculated by the estimation unit 152.

### (Communication unit 140)

The communication unit 140 is implemented by, for example, a network interface card (NIC) or the like. Then, the communication unit 140 is connected to the network in a wired or wireless manner to transmit and receive information to and from, for example, the application server 10 or the terminal device 20.

### (Control unit 150)

The control unit 150 is a controller, and is implemented by executing various programs (corresponding to an example of an information processing program) stored in the storage device provided in the information processing device 100, for example, by a central processing unit (CPU), micro processing unit (MPU), or the like, with the RAM as a working area. In addition, the control unit 150 is a controller, and is implemented by an integrated circuit such as an application specific integrated circuit (ASIC) or field programmable gate array (FPGA).

As illustrated in FIG. 3, the control unit 150 includes the sensor information processing unit 151, the estimation unit 152, the drive control unit 153, and a transmission unit 154, and implements or carries out the function of the information processing which is described below. Note that the internal configuration of the control unit 150 is not limited to the configuration illustrated in FIG. 3, and may have another configuration as long as information processing described later is performed.

### (Sensor information processing unit 151)

The sensor information processing unit 151 acquires raw data sensed by the finger sensor 110, from the finger sensor 110. The sensor information processing unit 151 performs sampling, noise reduction, filter application for a signal detected by the finger sensor 110, and performs conversion of a force detected by the finger sensor 110 to a voltage.

### (Estimation unit 152)

The estimation unit 152 acquires the sensor information from the finger sensor 110, from the sensor information processing unit 151. Subsequently, the estimation unit 152 having acquired the sensor information estimates the finger feature information on the basis of the acquired sensor information. Specifically, the estimation unit 152 estimates the finger feature information indicating the angle, average angular velocity, maximum angular velocity, and range of motion of each finger joint. Subsequently, upon estimating the finger feature information, the estimation unit 152 stores the estimated finger feature information in the storage unit 130.

Specifically, the estimation unit 152 acquires, from the finger sensor 110, the sensor information about the intensity of reflected light from the finger to which light is emitted from the photoreflector of the finger sensor 110. For example, the estimation unit 152 acquires the sensor information about the intensity of light reflected and returned from the surface of the skin of the distal phalanx to which infrared light is emitted from the photoreflector mounted on the extension of the upper surface of the middle phalanx. Alternatively, the estimation unit 152 acquires the sensor information about the distance between the sensor and the skin of the distal phalanx measured on the basis of the intensity of light. Subsequently, the estimation unit 152 calculates the angle θ₂ of the DIP joint, on the basis of the acquired sensor information. Subsequently, the estimation unit 152 estimates the angular velocity of the DIP joint by differentiating the angle θ₂ of the DIP joint with respect to time. Furthermore, the estimation unit 152 estimates the range of motion of the DIP joint on the basis of a maximum value and a minimum value of the angle θ₂ within a range of movement of the DIP joint during the sensing. As described above, the estimation unit (estimation unit 152) estimates the posture of the finger on the basis of the angles of the finger joints detected by the detection unit (photoreflectors).

In addition, the estimation unit 152 acquires the sensor information about the intensity of light reflected and returned from the surface of the skin of the proximal phalanx to which infrared light is emitted from the photoreflector mounted on the extension of the upper surface of the middle phalanx. Alternatively, the estimation unit 152 acquires the sensor information about the distance between the sensor and the skin of the proximal phalanx measured on the basis of the intensity of light. Subsequently, the estimation unit 152 calculates the angle θ₁ of the PIP joint, on the basis of the acquired sensor information. Subsequently, the estimation unit 152 estimates the angular velocity of the PIP joint by differentiating the angle θ₁ of the PIP joint with respect to time. Furthermore, the estimation unit 152 estimates the range of motion of the PIP joint by integrating the angle θ₁ of the PIP joint with respect to time.

Furthermore, the estimation unit 152 acquires the sensor information about the posture of the back of the hand (or the palm of the hand) detected by the 9-axis posture sensor attached to the back of the hand. Subsequently, the estimation unit 152 estimates the absolute angle α₀ of the palm of the hand relative to the horizontal plane, on the basis of the acquired sensor information.

In addition, the estimation unit 152 acquires the sensor information about the posture of the middle phalanx detected by the 9-axis posture sensor mounted to the middle phalanx. Subsequently, the estimation unit 152 estimates the absolute angle α₁ of the middle phalanx relative to the horizontal plane, on the basis of the acquired sensor information.

Furthermore, the estimation unit 152 calculates the angle θ₀ of the MP joint, on the basis of the estimated absolute angle α₀ of the palm of the hand and the absolute angle α₁ of the middle phalanx, the calculated angle θ₁ of the PIP joint, and the relational expression "α₀ - θ₀ - θ₁ = α₁" described with reference to FIG. 7. Subsequently, the estimation unit 152 estimates the angular velocity of the MP joint by differentiating the angle θ₀ of the MP joint with respect to time. Furthermore, the estimation unit 152 estimates the range of motion of the MP joint by integrating the angle θ₀ of the MP joint with respect to time.

Note that the estimation unit 152 may estimate the posture of the finger, on the basis of the state of the exoskeletal robot and the angles of the finger joints detected by the detection unit (photoreflectors). This configuration makes it possible for the information processing device 100 to more appropriately estimate the posture of the finger.

### (Drive control unit 153)

The drive control unit 153 refers to the storage unit 130 to acquire the finger feature information estimated by the estimation unit 152. Subsequently, upon acquiring the finger feature information, the drive control unit 153 generates the command to the exoskeletal robot 120 on the basis of the acquired information. Then, upon generating the command to the exoskeletal robot 120, the drive control unit 153 controls the drive of the exoskeletal robot 120 on the basis of the generated command.

### (Transmission unit 154)

The transmission unit 154 transmits the finger feature information estimated by the estimation unit 152 to application server 10. Specifically, in response to a request from the application server 10, the transmission unit 154 transmits the finger feature information estimated by the estimation unit 152. For example, the transmission unit 154 refers to the storage unit 130 and transmits the finger feature information indicating the angle, average angular velocity, maximum angular velocity, and range of motion of the DIP joint, the PIP joint, or the MP joint of the finger.

### [1.3. Operation example of information processing system]

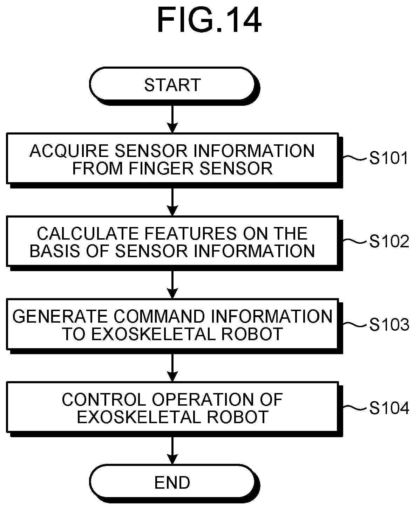

Next, an information processing procedure according to the first embodiment of the present disclosure will be described with reference to FIG. 14. FIG. 14 is a flowchart illustrating the information processing procedure according to the first embodiment of the present disclosure. As illustrated in FIG. 14, the information processing device 100 acquires the sensor information from the finger sensor 110 (Step S101). Next, the information processing device 100 calculates the finger features on the basis of the sensor information acquired from the finger sensor 110 (Step S102). Subsequently, upon calculating the finger features, the information processing device 100 generates command information to the exoskeletal robot 120 (Step S103). Then, upon generating the command information to the exoskeletal robot 120, the information processing device 100 controls the operation of the exoskeletal robot 120 on the basis of the generated command information (Step S104).

### [1.4. Modifications]

### [1.4.1. Finger sensor]

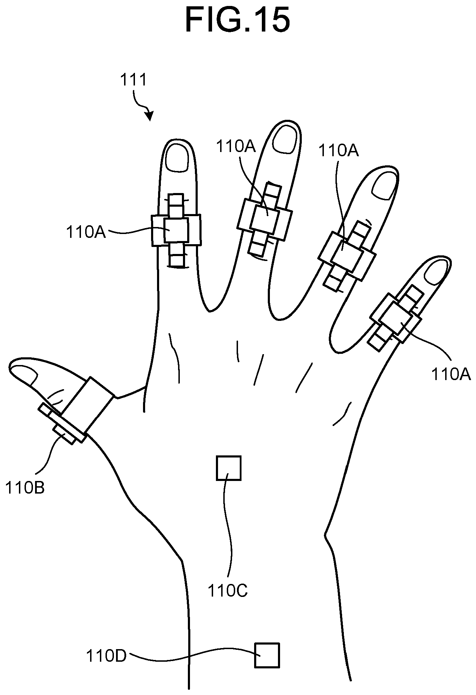

Next, a finger sensor according to a modification of the first embodiment of the present disclosure will be described with reference to FIG. 15. FIG. 15 is an exemplary view of the finger sensor according to the modification of the first embodiment of the present disclosure. A finger sensor 111 illustrated in FIG. 15 is different from the finger sensor 110 illustrated in FIG. 4 in that a 9-axis posture sensor 110D for measurement of wrist posture is further provided on the forearm. Use of the finger sensor 111 makes it possible to calculate, as the inclination of the wrist, a relative angle between the absolute angle of the back of the hand detected by the 9-axis posture sensor 110C for measurement of the reference posture of the back of the hand and an absolute angle of the wrist detected by the 9-axis posture sensor 110D for measurement of wrist posture.

As described above, the information processing device 100 further includes a third posture sensor (the 9-axis posture sensor 110D) for detecting the inclination of the wrist relative to the gravity direction. The third posture sensor is a 9-axis sensor that detects acceleration in the three-axis directions, angular velocity in the three-axis directions, and orientation in the three-axis directions. This configuration makes it possible for the information processing device 100 to appropriately detect the inclination of the wrist relative to the gravity direction. The estimation unit calculates, as an inclination angle of the wrist, an inclination of the wrist relative to the gravity direction with respect to the back of the hand, on the basis of the inclination of the wrist relative to the gravity direction detected by the third posture sensor and the inclination of the back of the hand relative to the gravity direction detected by the second posture sensor. This configuration makes it possible for the information processing device 100 to estimate the posture of the wrist, further improving accuracy in estimation of the postures of the fingers.

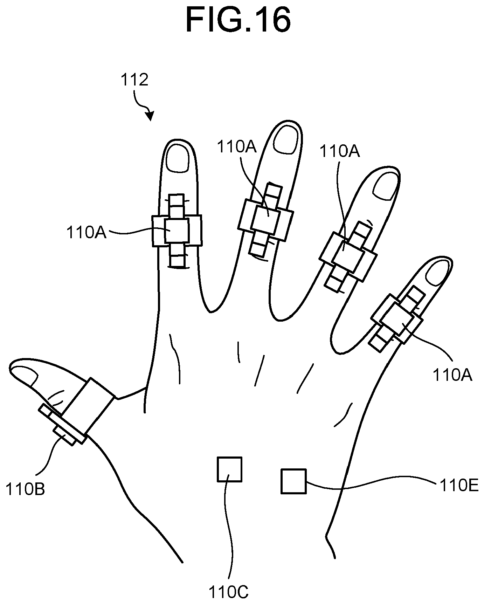

Next, a finger sensor according to a modification of the first embodiment of the present disclosure will be described with reference to FIG. 16. FIG. 16 is an exemplary view of the finger sensor according to the modification of the first embodiment of the present disclosure. A finger sensor 112 illustrated in FIG. 16 is different from the finger sensor 110 illustrated in FIG. 4 in that a 9-axis posture sensor 110E for measuring the curvature of the palm of the hand is further provided on the extension of the proximal phalanx bones of the ring finger and the little finger, on the back of the hand. Use of the finger sensor 112 makes it possible to calculate, as a degree of bending of the palm of the hand, a relative angle between the absolute angle of the back of the hand detected by the 9-axis posture sensor 110C for measurement of the reference posture of the back of the hand and an absolute angle of the curvature of the palm of the hand detected by the 9-axis posture sensor 110E for measuring the curvature of the palm of the hand. At this time, upon calculation of the stretching/bending angle of the respective joints of the ring finger and the little finger, use of an angle of the 9-axis posture sensor 110E for measurement of the palm of the hand relative to a horizontal plane, as α₀ described above makes it possible to increase the accuracy in calculation of the stretching/bending angle performed while curving the palm of the hand.

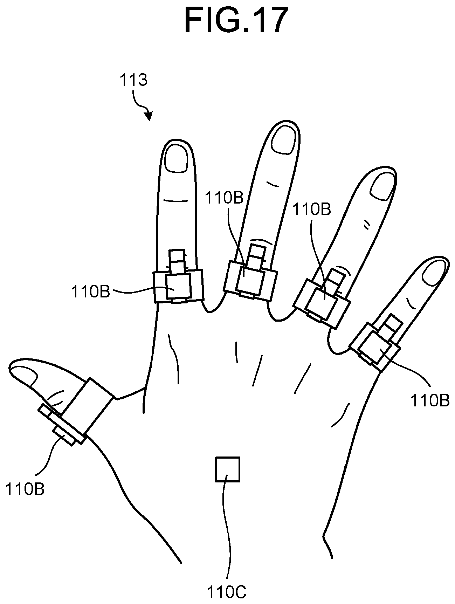

Next, a finger sensor according to a modification of the first embodiment of the present disclosure will be described with reference to FIG. 17. FIG. 17 is an exemplary view of the finger sensor according to the modification of the first embodiment of the present disclosure. A finger sensor 113 illustrated in FIG. 17 is different from the finger sensor 110 illustrated in FIG. 4 in that the second sensor unit 110B is attached to the proximal phalanx of each finger. Use of the finger sensor 113 makes it possible to perform measurement with less restriction although the DIP joint angle cannot be measured. In addition, use of the finger sensor 113 makes it possible to directly calculate the MP joint angle of each finger on the basis of a relative angle between the 9-axis posture sensor of each second sensor unit 110B and the 9-axis posture sensor 110C for measurement of the reference posture of the back of the hand.

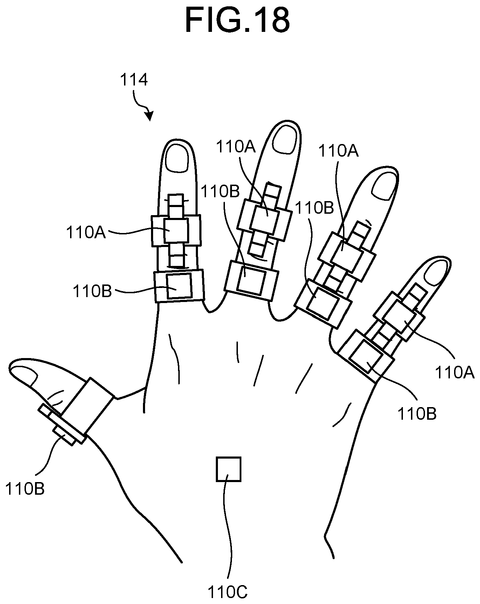

Next, a finger sensor according to a modification of the first embodiment of the present disclosure will be described with reference to FIG. 18. FIG. 18 is an exemplary view of the finger sensor according to the modification of the first embodiment of the present disclosure. A finger sensor 114 illustrated in FIG. 18 is different from the finger sensor 110 illustrated in FIG. 4 in that the second sensor unit 110B is attached to the proximal phalanx of each finger, in addition to the first sensor unit 110A. Use of the finger sensor 114 makes it possible to directly calculate the MP joint angle of each finger on the basis of the relative angle between the 9-axis posture sensor of each second sensor unit 110B and the 9-axis posture sensor 110C for measurement of the reference posture of the back of the hand.

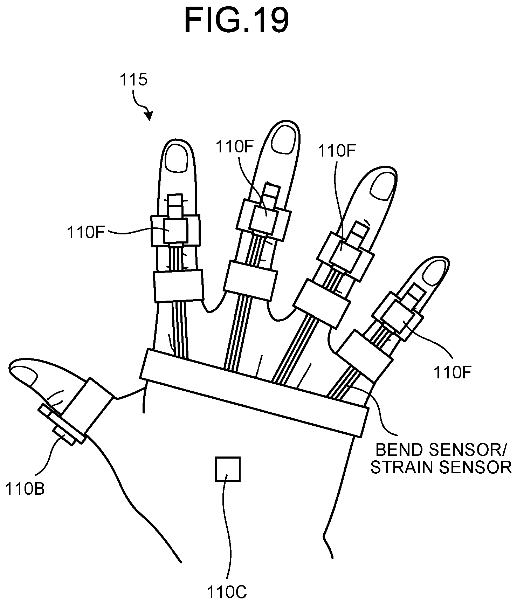

Next, a finger sensor according to a modification of the first embodiment of the present disclosure will be described with reference to FIG. 19. FIG. 19 is an exemplary view of the finger sensor according to the modification of the first embodiment of the present disclosure. A finger sensor 115 illustrated in FIG. 19 is different from the finger sensor 110 illustrated in FIG. 4 in that some of the sensors are replaced with a bend sensor, a strain sensor, or the like arranged across a joint of the finger to be measured. Use of the finger sensor 115 makes it possible to improve accuracy in measurement of the angle of the joint of the finger to be measured.

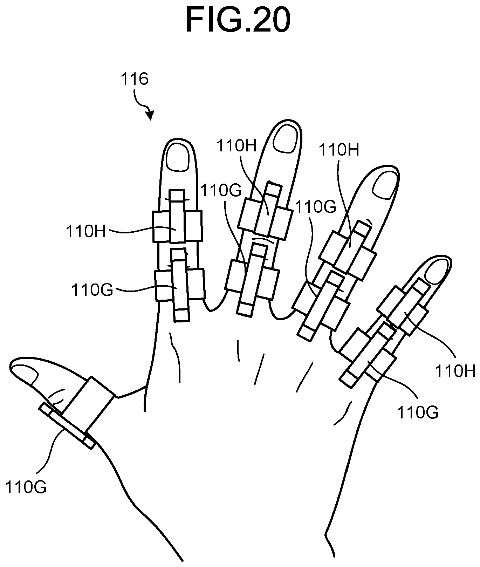

Next, a finger sensor according to a modification of the first embodiment of the present disclosure will be described with reference to FIG. 20. FIG. 20 is an exemplary view of the finger sensor according to the modification of the first embodiment of the present disclosure. A finger sensor 116 illustrated in FIG. 20 is different from the finger sensor 110 illustrated in FIG. 4 in that no 9-axis posture sensor is used and additional photoreflector is mounted on each MP joint. Use of the finger sensor 116 makes it possible to directly calculate the angles of all joints of each finger by measurement with the photoreflectors.

### [1.4.2. Exoskeletal robot]

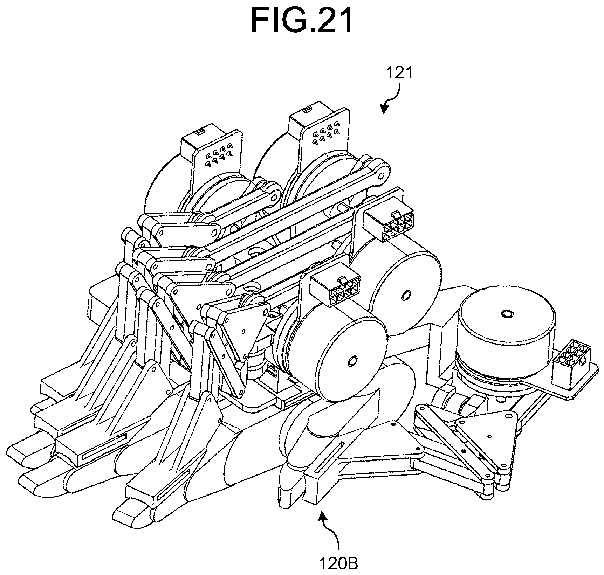

Next, an exoskeletal robot according to a modification of the first embodiment of the present disclosure will be described with reference to FIG. 21. FIG. 21 is a perspective view of the exoskeletal robot according to the modification of the first embodiment of the present disclosure. The exoskeletal robot illustrated in FIG. 21 is different from the exoskeletal robot 120 illustrated in FIG. 9 in that an exoskeleton unit corresponding to the thumb is added.

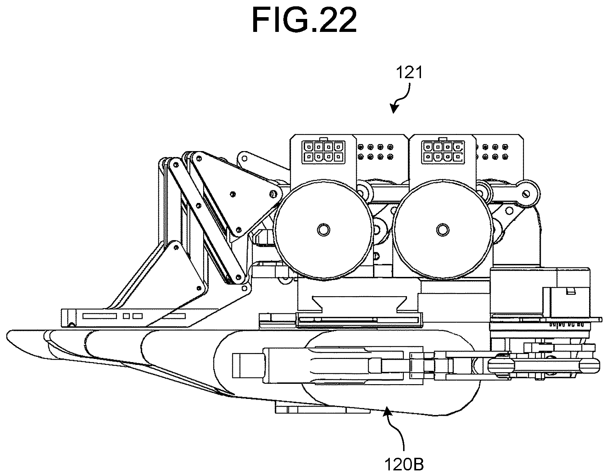

Next, the exoskeletal robot according to the modification of the first embodiment of the present disclosure will be further described with reference to FIG. 22. FIG. 22 is a side view of the exoskeletal robot according to the modification of the first embodiment of the present disclosure. FIG. 22 is a side view of the exoskeletal robot to which the exoskeleton unit corresponding to the thumb illustrated in FIG. 21 is added.

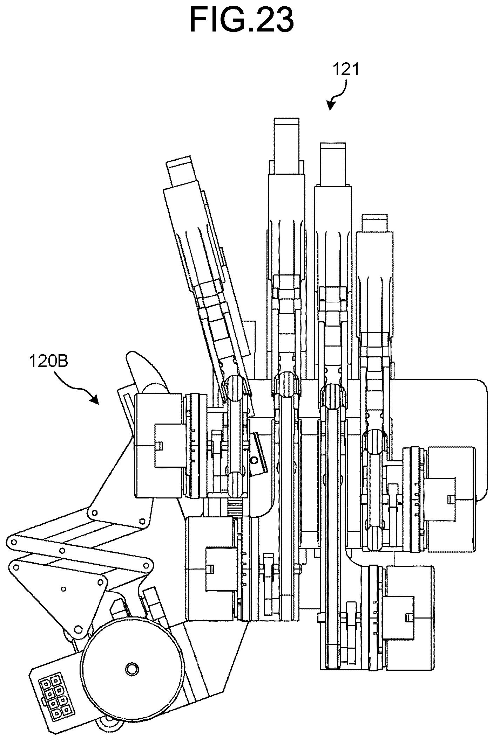

Next, the exoskeletal robot according to the modification of the first embodiment of the present disclosure will be further described with reference to FIG. 23. FIG. 23 is a top view of the exoskeletal robot according to the modification of the first embodiment of the present disclosure. FIG. 23 is a top view of the exoskeletal robot to which the exoskeleton unit corresponding to the thumb illustrated in FIG. 21 is added.

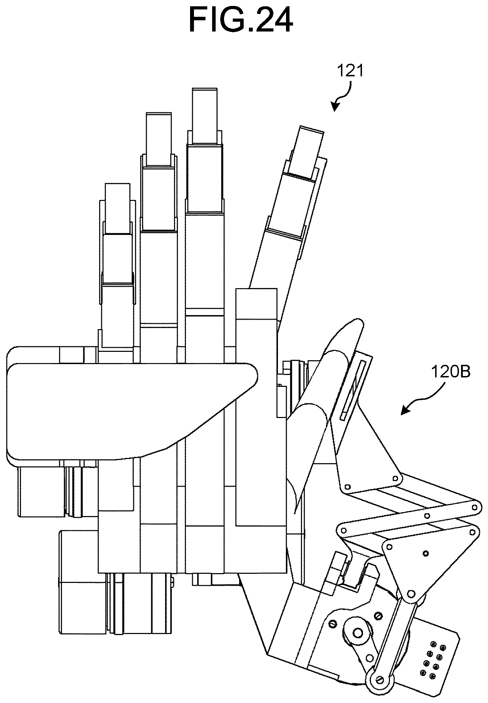

Next, the exoskeletal robot according to the modification of the first embodiment of the present disclosure will be further described with reference to FIG. 24. FIG. 24 is a bottom view of the exoskeletal robot according to the modification of the first embodiment of the present disclosure. FIG. 24 is a bottom view of the exoskeletal robot to which the exoskeleton unit corresponding to the thumb illustrated in FIG. 21 is added.

### [2. Example]

### [2.1. Overview of information processing system]

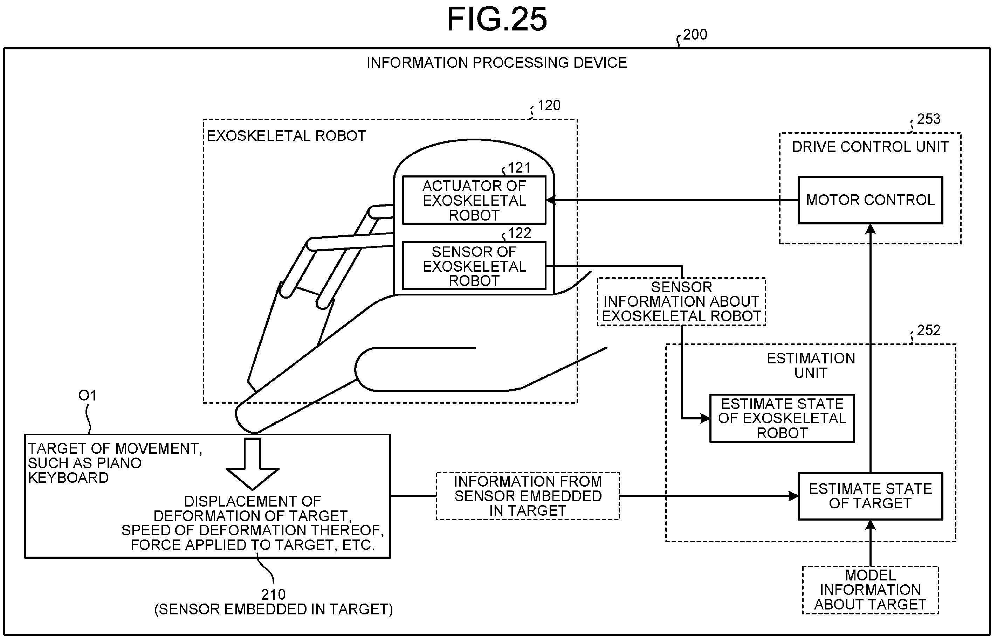

Next, an overview of information processing according to an example of the present disclosure not falling under the scope of the claims but useful for their understanding will be described with reference to FIG. 25. FIG. 25 is a diagram illustrating an example of the information processing according to the example of the present disclosure. In the example illustrated in FIG. 25, an information processing device 200 is different from the information processing device 100 of FIG. 1 in that a target sensor 210 that is embedded in a target O1 is provided instead of the finger sensor 110. Here, the target O1 refers to a target of finger movement. For example, when movements of the fingers represents playing piano, the target O1 of finger movement corresponds to a piano keyboard.

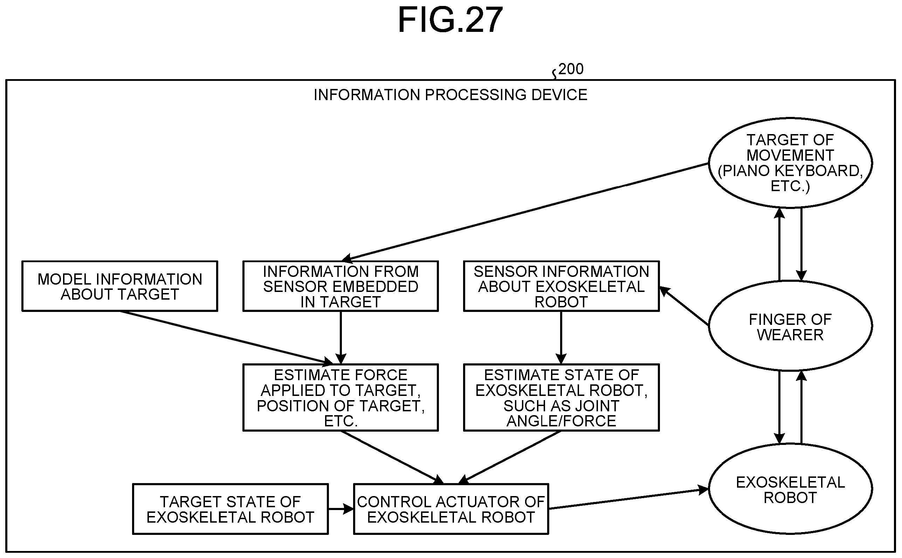

In order to control such an exoskeletal robot, sensors mounted to the exoskeletal robot itself are used generally, but for skill learning support or the like, it may be possible to embed the sensors in the target of finger movement, in some cases. In addition, in a case where the target is controlled by the exoskeletal robot through the fingers of the wearer, the model of the fingers of the wearer is interposed between the target and an output from the exoskeletal robot, as illustrated in FIG. 27 which is described later, and therefore, appropriate control is sometimes made difficult.

Therefore, in the information processing system according to the example of the present disclosure, an information processing system 2 is proposed that uses sensor information from a sensor embedded in the target of finger movement to control an exoskeletal robot safely with higher accuracy. The information processing system 2 also feeds back information from the sensor embedded in the target to the exoskeletal robot, for an attempt to provide more accurate control. Specifically, stable control is performed by running a control loop illustrated in FIG. 25 at a high speed of 100 Hz or more. Note that some control loops, such as a control loop in a exoskeletal robot 220, may be made faster. Note that the information processing system 2 is different from the information processing system 1 illustrated in FIG. 2 in that the information processing device 200 is provided instead of the information processing device 100, but otherwise has the same configuration as the information processing system 1.

In the example illustrated in FIG. 25, the information processing device 200 has an estimation unit 252 that acquires the sensor information from the target sensor 210. For example, the estimation unit 252 acquires the sensor information indicating displacement of deformation of the target O1 caused by pressing the target O1 by the finger of the wearer wearing the exoskeletal robot 220, the speed of the deformation, a force applied to the target, and the like. Furthermore, the estimation unit 252 acquires model information about the target O1. For example, in a case where the target O1 is the piano keyboard, the model information about the target O1 represents information about a model having the shape of the piano keyboard. Subsequently, upon acquiring the sensor information and the model information, the estimation unit 252 estimates the state of the target O1 on the basis of the acquired sensor information and model information. Subsequently, upon estimating the state of the target O1, the estimation unit 252 outputs information about the state of the target O1 estimated to a drive control unit 253.

Furthermore, the estimation unit 252 of the information processing device 200 acquires sensor information about the exoskeletal robot 120 from the sensor 122 of the exoskeletal robot 120. Subsequently, upon acquiring the sensor information about the exoskeletal robot 120, the estimation unit 252 estimates the state of the exoskeletal robot 120 on the basis of the acquired sensor information. Subsequently, upon estimating the state of the exoskeletal robot 120, the estimation unit 252 outputs information about the state of the exoskeletal robot 120 estimated to the drive control unit 253.

The drive control unit 253 of the information processing device 200 acquires the information about the state of the target O1 and the information about the state of the exoskeletal robot 120, from the estimation unit 252. Subsequently, upon acquiring the information about the state of the target O1 and the information about the state of the exoskeletal robot 120, the drive control unit 253 controls the drive of the drive unit 121 (e.g., motor) of the exoskeletal robot 120, on the basis of the acquired information about the state of the target O1 and information about the state of the exoskeletal robot 120.

### [2.2. Configuration example of information processing device]

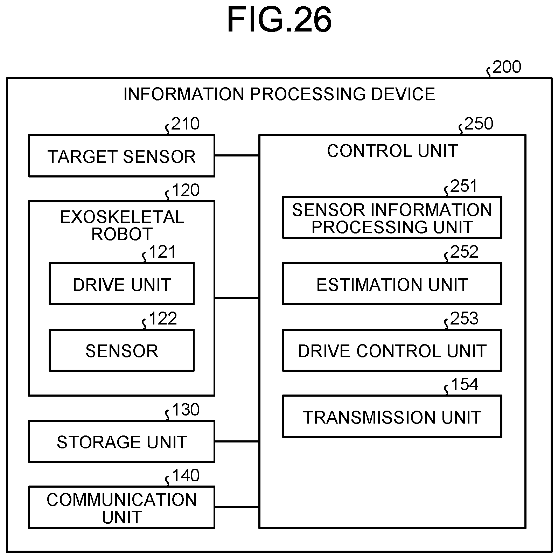

Next, a configuration of the information processing device according to the example of the present disclosure will be described with reference to FIG. 26. FIG. 26 is a diagram illustrating a configuration example of the information processing device according to the example of the present disclosure. As illustrated in FIG. 26, the information processing device 200 according to the example includes the exoskeletal robot 120, the storage unit 130, and the communication unit 140, as in the information processing device 100 according to the first embodiment. Furthermore, the information processing device 200 is different from the information processing device 100 in that the target sensor 210 and a control unit 250 are provided. Note that, hereinafter, description of portions overlapping with the information processing device 100 will be appropriately omitted.

### (Target sensor 210)

The target sensor 210 is a sensor embedded in the target O1 of finger movement. For example, the target sensor 210 is implemented by a contact sensor mounted in the target. For example, the target sensor 210 detects the displacement of deformation of the target O1 caused by pressing the target O1 by the finger of the wearer wearing the exoskeletal robot 220, the speed of the deformation, a force applied to the target, and the like.

### (Control unit 250)

The control unit 250 is a controller, and is implemented by executing various programs (corresponding to an example of the information processing program) stored in the storage device provided in the information processing device 100, for example, by CPU, MPU, or the like, with the RAM as a working area. In addition, the control unit 250 is a controller, and is implemented by an integrated circuit such as ASIC or FPGA.

As illustrated in FIG. 26, the control unit 250 includes a sensor information processing unit 251, the estimation unit 252, the drive control unit 253, and the transmission unit 154, and implements or carries out the function of the information processing which is described below. Note that the internal configuration of the control unit 250 is not limited to the configuration illustrated in FIG. 26, and may have another configuration as long as information processing described later is performed.

### (Sensor information processing unit 251)

The sensor information processing unit 251 acquires raw data sensed by the target sensor 210, from the target sensor 210. The sensor information processing unit 251 performs sampling, noise reduction, and filter application for a signal detected by the target sensor 210, and performs conversion of a force detected by the target sensor 210 to a voltage.

### (Estimation unit 252)

The estimation unit 252 acquires the sensor information detected by the target sensor 210, from the sensor information processing unit 251. For example, the estimation unit 252 acquires the sensor information indicating the displacement of deformation of the target O1 caused by pressing the target O1 by the finger of the wearer wearing the exoskeletal robot 220, the speed of the deformation, a force applied to the target, and the like. Furthermore, the estimation unit 252 acquires the model information about the target O1. Subsequently, upon acquiring the sensor information and the model information, the estimation unit 252 estimates the state of the target O1 on the basis of the acquired sensor information and model information. Subsequently, upon estimating the state of the target O1, the estimation unit 252 outputs information about the state of the target O1 estimated to a drive control unit 253.

In addition, the estimation unit 252 acquires the sensor information about the exoskeletal robot 120 from the sensor 122 of the exoskeletal robot 120. Subsequently, upon acquiring the sensor information about the exoskeletal robot 120, the estimation unit 252 estimates the state of the exoskeletal robot 120 on the basis of the acquired sensor information. For example, the estimation unit 252 estimates information about a force and speed applied by the exoskeletal robot 120, and a force and speed given by the wearer of the exoskeletal robot 120. Subsequently, upon estimating the state of the exoskeletal robot 120, the estimation unit 252 outputs information about the state of the exoskeletal robot 120 estimated to the drive control unit 253.

### (Drive control unit 253)

The drive control unit 253 acquires the information about the state of the target O1 and the information about the state of the exoskeletal robot 120, from the estimation unit 252. Subsequently, upon acquiring the information about the state of the target O1 and the information about the state of the exoskeletal robot 120, the drive control unit 253 recalculates output from each motor 121A relative to a target force and position, on the basis of the acquired information about the state of the target O1 and information about the state of the exoskeletal robot 120. Subsequently, upon recalculating the output from the motor 121A, the drive control unit 253 controls the drive of the motor 121A on the basis of the recalculated output.

### [2.3. Operation example of information processing system]

Next, the concept of a process of information processing according to the example of the present disclosure will be described with reference to FIG. 27. FIG. 27 is a conceptual diagram illustrating the process of information processing according to the example of the present disclosure. As illustrated in FIG. 27, a force is physically applied and received, between the target of movement such as the piano keyboard according to the example and the fingers of the wearer. Likewise, a force is physically applied and received, between the fingers of the wearer and the exoskeletal robot.

Furthermore, the estimation unit 252 estimates the state of the target such as s force applied to the target and the position of the target, on the basis of the sensor information from the target sensor embedded in the target and the model information about the target. In addition, the estimation unit 252 estimates the state of the exoskeletal robot, such as the angle of the joint and force of the exoskeletal robot, on the basis of the sensor information about the exoskeletal robot.

Next, the drive control unit 253 controls the drive of an actuator of the exoskeletal robot, on the basis of a target state of the exoskeletal robot acquired in advance, and the state of the target and the state of the exoskeletal robot which are estimated by the estimation unit 252. For example, the drive control unit 253 controls the drive of each motor of the exoskeletal robot. Furthermore, the drive control unit 253 controls the drive of the motor to control each link unit of the exoskeletal robot.

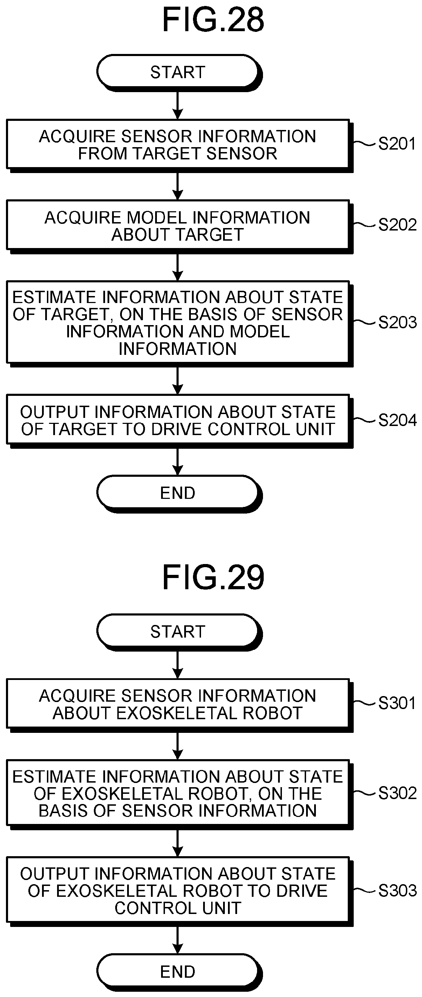

Next, an information processing procedure according to the example of the present disclosure will be described with reference to FIG. 28. FIG. 28 is a flowchart illustrating the information processing procedure according to the example of the present disclosure. As illustrated in FIG. 28, the estimation unit 252 acquires the sensor information from the target sensor 210 (Step S201). Subsequently, the estimation unit 252 acquires the model information about the target O1 (Step S202). Next, the estimation unit 252 estimates the information about the state of the target O1 on the basis of the sensor information and the model information (Step S203). Then, the estimation unit 252 outputs the information about the state of the target O1 to the drive control unit 253 (Step S204).

Next, an information processing procedure according to the example of the present disclosure will be described with reference to FIG. 29. FIG. 29 is a flowchart illustrating the information processing procedure according to the example of the present disclosure. As illustrated in FIG. 29, the estimation unit 252 acquires sensor information about the exoskeletal robot 220 (Step S301). Subsequently, the estimation unit 252 estimates the information about the state of the exoskeletal robot 220 on the basis of the sensor information (Step S302). Then, the estimation unit 252 outputs the information about the state of the exoskeletal robot 220, to the drive control unit 253 (Step S303).

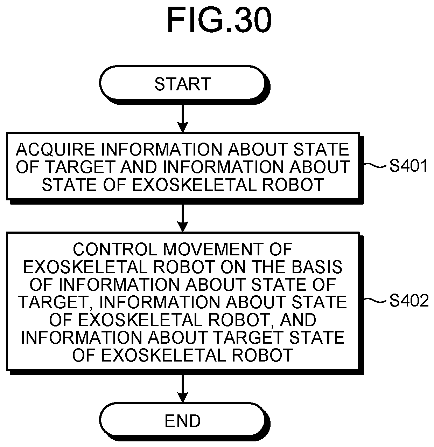

Next, an information processing procedure according to the example of the present disclosure will be described with reference to FIG. 30. FIG. 30 is a flowchart illustrating the information processing procedure according to the example of the present disclosure. As illustrated in FIG. 30, the drive control unit 253 acquires the information about the state of the target O1 and the information about the state of the exoskeletal robot 220, from the estimation unit 252 (Step S401). Subsequently, the drive control unit 253 controls the movement of the exoskeletal robot 220, on the basis of the information about the state of the target O1, the information about the state of the exoskeletal robot 220, and information about the target state of the exoskeletal robot 220 (Step S402).

### [3. Others]

Next, an example of a screen display on a terminal device of the user with a result of the processing according to the example displayed will be described with reference to FIG. 31. FIG. 31 is an exemplary view of the screen display on the terminal device of the user with a result of the processing according to the example displayed. In the example illustrated in FIG. 31, the terminal device 20 displays information about the postures and positions of the fingers of the user estimated by the information processing device 100 and information about the postures and positions of the fingers serving as a model, in a comparable manner. In FIG. 31, an image of a hand indicated by "You" on the left side of the screen display on the terminal device 20 corresponds to the information about the postures and positions of the fingers of the user. Furthermore, an image of a hand indicated by "Teacher" on the right side of the screen display on the terminal device 20 corresponds to the information about the postures and positions of the fingers serving as the model. Furthermore, the terminal device 20 may display the information about the postures and positions of the fingers of the user estimated by the information processing device 100 and information about the postures and positions of the fingers of the user in the past, in a comparable manner. For example, the terminal device 20 displays an image relating to the postures and positions of the fingers of the user estimated by the information processing device 100 on the left side of the screen display, and displays an image relating to the postures and positions of the fingers of the user in the past on the right side of the screen display. Furthermore, the terminal device 20 may display moving images of moving fingers in a comparable manner, or may display still images of the fingers at a certain moment in a comparable manner. In this manner, the terminal device 20 displays the information about the postures and positions of the fingers of the user estimated by the information processing device 100.

Next, an example of a screen display on the terminal device of the user with a result of the processing according to the example displayed will be described with reference to FIG. 32. FIG. 32 is an exemplary view of the screen display on the terminal device of the user with a result of the processing according to the example displayed. In the example illustrated in FIG. 32, for example, the terminal device 20 displays, as a moving image, a difference between the movements of the fingers of the user estimated by the information processing device 100 and the movements of the fingers serving as the model. Furthermore, the terminal device 20 displays three-dimensional features such as the speed of fingertips of the user estimated by the information processing device 100 and three-dimensional features such as the speed of fingertips serving as a model, in a comparable manner. Furthermore, the terminal device 20 may display, as the moving image, a difference between the movements of the fingers of the user estimated by the information processing device 100 and the movement of the fingers of the user in the past. For example, the terminal device 20 displays the three-dimensional features such as the speed of fingertips of the user estimated by the information processing device 100 and three-dimensional features such as the speed of fingertips of the user in the past, in a comparable manner. Furthermore, the terminal device 20 may be augmented reality (AR) glasses or a head mounted display (HMD). The user wears the terminal device 20 that is the AR glass or HMD. Then, the terminal device 20 displays an image indicating the movements of the fingers serving as the model or the movements of the fingers of the user in the past, over the fingers of the user actually moving by using AR. As a result, the terminal device 20 is allowed to more specifically present information to the user.

### [4. Effects]

As described above, the information processing devices according to the first embodiment, the example, and the modifications each include the detection unit and the estimation unit. The detection unit detects the angles of at least some of the joints of each finger, in a non-contact manner. The estimation unit estimates the posture of the finger on the basis of the angles of the joints of the finger detected by the detection unit.

Therefore, the information processing device performs detection in the non-contact manner, thus making it possible to detect the finger joint angles without hindering the sensitivity of the fingertips and the fine movements of the fingers. Therefore, the information processing device is configured to appropriately measure the movements of the fingers.

In addition, the detection unit includes the non-contact sensor, detects the distance from the non-contact sensor to each finger that changes according to the angle of each finger joint, and thus detects the angle of the finger joint on the basis of the detected distance. In addition, the non-contact sensor includes a light emitting element that emits light and a light receiving element that detects reflected light of the light. The light emitting element emits light to the finger joint. The light receiving element detects reflected light of the light emitted to a finger phalanx. The detection unit detects the distance to the finger on the basis of the intensity of the reflected light detected by the light receiving element.

Therefore, in the information processing device, detection of the distance from the non-contact sensor to the finger, instead of direct detection of the angle of each joint by mounting a sensor to each joint, makes it possible to indirectly (i.e., in a non-contact manner) detect the angle of the joint.

Furthermore, the non-contact sensor is provided at the middle phalanx of at least one of the index finger, the middle finger, the ring finger, and the little finger. The light emitting element emits light to at least one of the distal phalanx and the proximal phalanx of the finger. The light receiving element detects the reflected light of the light emitted to at least one of the distal phalanx and the proximal phalanx of the finger. The detection unit calculates the relative angle of the distal phalanx of the finger to the middle phalanx of the finger, as the DIP joint angle of the finger, on the basis of the distance between the distal phalanx of the finger and the non-contact sensor calculated on the basis of the intensity of the reflected light. The estimation unit calculates the relative angle of the proximal phalanx of the finger to the middle phalanx of the finger, as the PIP joint angle of the finger, and estimates the posture of the finger on the basis of the DIP joint angle and the PIP joint angle of the finger.

This configuration makes it possible for the information processing device to detect the DIP joint angle and the PIP joint angle of the finger in the non-contact manner without mounting a sensor to the distal phalanx and the proximal phalanx of the finger.

Furthermore, the information processing device further includes the first posture sensor for detecting the inclination of the middle phalanx of the finger relative to the gravity direction. The estimation unit calculates the MP joint angle of the finger, on the basis of the PIP joint angle of the finger and the inclination of the middle phalanx of the finger relative to the gravity direction that is detected by the first posture sensor. Furthermore, the information processing device further includes the second posture sensor for detecting the inclination of the back of the hand relative to the gravity direction. The estimation unit calculates, as the MP joint angle, a relative angle of the proximal phalanx of the finger to the back of the hand, on the basis of the inclination of the back of the hand relative to the gravity direction, detected by the second posture sensor.

Therefore, this configuration makes it possible for the information processing device to calculate the MP joint angle in the non-contact manner without mounting a sensor to the MP joint.

In addition, the second posture sensor is provided in the area between the MP joints on the proximal phalanx bones of the index finger and the middle finger and the wrist of the back of the hand.

This configuration makes it possible for the information processing device to detect inclination of the back of the hand relative to the gravity direction so as not to be affected by the bending of the palm of the hand or the movement of the thumb.

Furthermore, the information processing device further includes the third posture sensor for detecting the inclination of the wrist relative to the gravity direction. The estimation unit calculates, as the inclination angle of the wrist, the inclination of the wrist relative to the gravity direction with respect to the back of the hand, on the basis of the inclination of the wrist relative to the gravity direction detected by the third posture sensor and the inclination of the back of the hand relative to the gravity direction detected by the second posture sensor.

This configuration makes it possible for the information processing device to estimate the posture of the wrist, further improving accuracy in estimation of the posture of the fingers.

In addition, the first posture sensor detects the inclination of the adduction or abduction of the finger relative to the gravity direction. The second posture sensor detects the inclination of the adduction or abduction of the palm of the hand relative to the gravity direction. The estimation unit calculates information about the relative angle of the adduction or abduction of the finger to the back of the hand, on the basis of the inclination of the adduction or abduction of the finger relative to the gravity direction detected by the first posture sensor and the inclination of the adduction or abduction of the palm of the hand relative to the gravity direction detected by the second posture sensor.

This configuration makes it possible for the information processing device to estimate the angle of the adduction or abduction of the finger with relatively low constraint.

Furthermore, the first posture sensor is an 9-axis sensor. Furthermore, the second posture sensor is an 9-axis sensor. Furthermore, the third posture sensor is an 9-axis sensor.

This configuration makes it possible for the information processing device to appropriately detect the inclination of the middle phalanx of the finger relative to the gravity direction, the inclination of the back of the hand relative to the gravity direction, and the inclination of the wrist relative to the gravity direction.

Furthermore, the information processing device further includes the exoskeletal robot, and the exoskeletal robot includes the remote center of motion (RCM) mechanisms each of which has the double parallelogram structure using the parallel links, the rotation mechanisms each of which rotates the RCM mechanism according to the rotation of the adduction or abduction of the finger, the holding members each of which is fixed to the first vertex diagonally positioned to the connection point of the two parallelogram structures of the RCM mechanism to hold the finger, the first links each of which has one end fixed to the second vertex diagonally positioned to the connection point, the second links each of which has one end connected to the other end of the first link by the first ball joint, and the third links each of which has one end connected to the other end of the second link by the second ball joint, in which the first link, the second link, and the third link constitute the four-bar link structure. Furthermore, the exoskeletal robot further includes the motors, and the other end of each third link is fixed to the rotation shaft of each of the motors.

Therefore, in the information processing device, the frictional resistance of the drive transmission unit of the exoskeletal robot can be reduced and the degrees of freedom of the fingers can be improved in the adduction or abduction, and thus, it is possible to reproduce smoother and more natural movements of the fingers for the wearer of the exoskeletal robot. Therefore, the information processing device is configured to appropriately reproduce the measured movements of the fingers to the user after appropriate measurement of the movements of the fingers.

In addition, the rotation axis of the rotation mechanism and the rotation axis of the RCM mechanism are arranged in the order of the rotation axis of the rotation mechanism and the rotation axis of the RCM mechanism, from near the wrist of the wearer of the exoskeletal robot. In addition, the rotation axis of the rotation mechanism is positioned at the MP joint of the finger of the wearer of the exoskeletal robot, and the rotation axis of the RCM mechanism is positioned within the predetermined range from the MP joint of the finger, in a direction viewing the PIP joint from the MP joint of the finger of the wearer of the exoskeletal robot.

This configuration makes it possible for the information processing device having arrangement of the rotation axis having the degree of freedom in the adduction or abduction of the finger and the RCM mechanism in this order, as viewed from the wrist side, to guide the finger as in the original movements of the finger. In other words, it is possible for the information processing device to guide the movement of the adduction or abduction of the finger based on the MP joint of the finger and the movements of bending and stretching the finger based on the MP joint, the PIP joint, and the DIP joint, in the same manner as the original movements of the finger.

Furthermore, the estimation unit may estimate the posture of the finger, on the basis of the state of the exoskeletal robot and the angles of the finger joints detected by the detection unit.

This configuration makes it possible for the information processing device to more appropriately estimate the posture of the finger.

### [5. Hardware configuration]

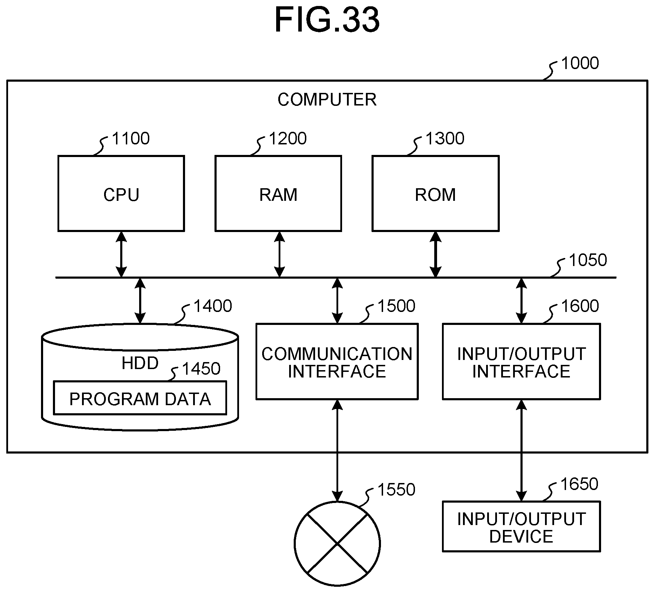

The information devices, such as the information processing device 100, according to the embodiments and modifications described above, are implemented by, for example, a computer 1000 having a configuration as illustrated in FIG. 33. FIG. 33 is a hardware configuration diagram illustrating an example of the computer 1000 implementing the functions of the information processing devices such as the information processing device 100. An example of the information processing device 100 according to the embodiment or modifications thereof which have been described above will be described below. The computer 1000 includes a CPU 1100, a RAM 1200, a read only memory (ROM) 1300, a hard disk drive (HDD) 1400, a communication interface 1500, and an input/output interface 1600. The respective units of the computer 1000 are connected by a bus 1050.

The CPU 1100 is operated on the basis of programs stored in the ROM 1300 or the HDD 1400 and controls the respective units. For example, the CPU 1100 deploys the programs stored in the ROM 1300 or the HDD 1400 to the RAM 1200 and executes processing corresponding to various programs.

The ROM 1300 stores a boot program, such as a basic input output system (BIOS), executed by the CPU 1100 when the computer 1000 is booted, a program depending on the hardware of the computer 1000, and the like.

The HDD 1400 is a computer-readable recording medium that non-transitorily records the programs executed by the CPU 1100, data used by the programs, and the like. Specifically, the HDD 1400 is a recording medium that records the information processing program according to an embodiment or a modification thereof of the present disclosure, the information processing program being an example of program data 1350.

The communication interface 1500 is an interface for connecting the computer 1000 to an external network 1550 (e.g., the Internet). For example, the CPU 1100 receives data from another device or transmits data generated by the CPU 1100 to another device, via the communication interface 1500.

The input/output interface 1600 is an interface for connecting an input/output device 1650 and the computer 1000. For example, the CPU 1100 receives data from an input device such as a keyboard or mouse via the input/output interface 1600. In addition, the CPU 1100 transmits data to an output device such as a display, speaker, or printer via the input/output interface 1600. Furthermore, the input/output interface 1600 may function as a media interface that reads a program or the like recorded on a predetermined recording medium. The medium includes, for example, an optical recording medium such as a digital versatile disc (DVD) or phase change rewritable disk (PD), a magneto-optical recording medium such as a magneto-optical disk (MO), a tape medium, a magnetic recording medium, a semiconductor memory, or the like.

For example, when the computer 1000 functions as the information processing device 100 according to the embodiment or modifications thereof, the CPU 1100 of the computer 1000 implements the functions of the control unit 150 and the like by executing the information processing program loaded on the RAM 1200. Furthermore, the HDD 1400 stores the information processing program according to an embodiment or modifications thereof of the present disclosure, or data stored in the storage unit 130. Note that the CPU 1100 executes the program data 1350 read from the HDD 1400, but in another example, the CPU 1100 may acquire the programs from another device via the external network 1550.

### Reference Signs List

- 1: INFORMATION PROCESSING SYSTEM

- 10: APPLICATION SERVER
- 20: TERMINAL DEVICE
- 100: INFORMATION PROCESSING DEVICE
- 110: FINGER SENSOR
- 120: EXOSKELETAL ROBOT
- 121: DRIVE UNIT
- 122: SENSOR
- 130: STORAGE UNIT
- 140: COMMUNICATION UNIT
- 150: CONTROL UNIT
- 151: SENSOR INFORMATION PROCESSING UNIT
- 152: ESTIMATION UNIT
- 153: DRIVE CONTROL UNIT
- 154: TRANSMISSION UNIT

## Claims

1. An information processing device (100) comprising:
a detection unit (110) configured to detect angles of at least some of joints of a finger in a non-contact manner; and
an estimation unit (152) configured to estimate a posture of the finger based on the angles of the joints detected by the detection unit.

2. The information processing device (100) according to claim 1, wherein
the detection unit (110) includes a non-contact sensor, is configured to detect a distance from the non-contact sensor to the finger, the distance changing according to the angles of the joints, and is configured to detect the angles of the joints based on the distance detected.

3. The information processing device (100) according to claim 2, wherein
the non-contact sensor includes a light emitting element configured to emit light and a light receiving element configured to detect reflected light of the light,
the light emitting element is configured to emit light to a phalanx of the finger,
the light receiving element is configured to detect reflected light of the light emitted to the phalanx of the finger, and
the detection unit (110) is configured to
detect the distance to the finger based on intensity of the reflected light detected by the light receiving element.

4. The information processing device (100) according to claim 3, wherein
the non-contact sensor is configured to be provided at a middle phalanx of at least one of an index finger, a middle finger, a ring finger, and a little finger, and
the light emitting element is configured to emit light to at least one of a distal phalanx and a proximal phalanx of the finger,
the light receiving element is configured to detect reflected light of the light emitted to at least one of the distal phalanx and the proximal phalanx of the finger,
the detection unit (110) is configured to
calculate, as an angle of a DIP joint of the finger, a relative angle of the distal phalanx of the finger to the middle phalanx of the finger, based on a distance between the distal phalanx of the finger and the non-contact sensor calculated based on the intensity of the reflected light; and
the estimation unit (152)
is configured to calculate, as an angle of a PIP joint of the finger, a relative angle of the proximal phalanx of the finger to the middle phalanx of the finger, and configured to estimate the posture of the finger based on the angle of the DIP joint and the angle of the PIP joint of the finger.

5. The information processing device (100) according to claim 4, further comprising
a first posture sensor (110A) configured to detect an inclination of the middle phalanx of the finger relative to a gravity direction,
wherein the estimation unit (152) is configured to
calculate an angle of an MP joint of the finger, based on the angle of the PIP joint of the finger and the inclination of the middle phalanx of the finger relative to the gravity direction detected by the first posture sensor (110A).

6. The information processing device (100) according to claim 5, further comprising
a second posture sensor (110C) configured to detect an inclination of the back of a hand relative to a gravity direction,
wherein the estimation unit (152) is configured to
calculate, as the angle of the MP joint, a relative angle of the proximal phalanx of the finger to the back of the hand, based on an inclination of the back of the hand relative to the gravity direction detected by the second posture sensor (110C).

7. The information processing device (100) according to claim 6, wherein
the second posture sensor is configured to be provided in an area between the MP joints on proximal phalanx bones of the index finger and the middle finger and a wrist on the back of the hand; or wherein
the first posture sensor (110A) is configured to detect an inclination of adduction or abduction of the finger relative to the gravity direction,
the second posture sensor (110C) is configured to detect an inclination of adduction or abduction of a palm relative to the gravity direction, and
the estimation unit (152) is configured to
calculate information about a relative angle of adduction or abduction of the finger to the back of the hand based on the inclination of adduction or abduction of the finger relative to the gravity direction detected by the first posture sensor (110A) and the inclination of adduction or abduction of the palm relative to the gravity direction detected by the second posture sensor (110C).

8. The information processing device (100) according to claim 6, further comprising
a third posture sensor (110D) configured to detect an inclination of a wrist relative to the gravity direction,
wherein the estimation unit (152) is configured to
calculate, as an inclination angle of the wrist, an inclination of the wrist relative to the gravity direction with respect to the back of the hand, on the basis of the inclination of the wrist relative to the gravity direction detected by the third posture sensor (110D) and the inclination of the back of the hand relative to the gravity direction detected by the second posture sensor (110C).

9. The information processing device (100) according to any one of claims 5 to 8, wherein
the first posture sensor (110A) is a 9-axis sensor.

10. The information processing device (100) according to any one of claims 6 to 8, wherein
the second posture sensor (110C) is a 9-axis sensor.

11. The information processing device (100) according to claim 8, wherein
the third posture sensor (110D) is a 9-axis sensor.

12. The information processing device (100) according to any one of claims 1 to 11, further comprising:
an exoskeletal robot (120) configured to reproduce the posture of the finger estimated by the estimation unit including:
a remote center of motion mechanism that has a double parallelogram structure using parallel links;
a rotation mechanism is configured to rotate the remote center of motion mechanism according to rotation of adduction or abduction of the finger;
a holding member that is fixed to a first vertex diagonally positioned to a connection point of two parallelogram structures of the remote center of motion mechanism and configured to hold the finger;
a first link (L1) having one end fixed to a second vertex (P2) diagonally positioned to the connection point;
a second link (L2) having one end connected to another end of the first link (L1) by a first ball joint (J1); and
a third link (L3) having one end connected to another end of the second link (L2) by a second ball joint (J2);
wherein the first link (L1), the second link (L2), and the third link (L3) constitute a four-bar link structure.

13. The information processing device (100) according to claim 12, wherein
the exoskeletal robot (120) further includes a motor (121A), and the third link (L3) has another end that is fixed to a rotation shaft of the motor (121A); and/or wherein
a rotation axis of the rotation mechanism and a rotation axis of the remote center of motion mechanism are arranged in an order of the rotation axis of the rotation mechanism and the rotation axis of the remote center of motion mechanism, from near a wrist of a wearer of the exoskeletal robot; and/or wherein
a rotation axis of the rotation mechanism is positioned at an MP joint of a finger of a wearer of the exoskeletal robot, and a rotation axis of the remote center of motion mechanism is positioned within a predetermined range from the MP joint of the finger, in a direction viewing a PIP joint from the MP joint of the finger of the wearer of the exoskeletal robot.

14. An information processing method comprising:
detecting, by a detection unit (110), angles of at least some of joints of a finger in a non-contact manner; and
estimating a posture of the finger based on the detected angles of the joints.

15. A computer program comprising instructions to cause the information processing device of claim 1 to execute the information processing method of claim 14.

## Patentansprüche

1. Informationsverarbeitungsvorrichtung (100), umfassend:
eine Erkennungseinheit (110), die konfiguriert ist, um Winkel von mindestens einigen Gelenken eines Fingers berührungslos zu erkennen, und
eine Schätzungseinheit (152), die konfiguriert ist, um eine Haltung des Fingers basierend auf den Winkeln der von der Erkennungseinheit erkannten Gelenke zu schätzen.

2. Informationsverarbeitungsvorrichtung (100) nach Anspruch 1, wobei
die Erkennungseinheit (110) einen berührungslosen Sensor einschließt, konfiguriert ist, um einen Abstand von dem berührungslosen Sensor zum Finger zu erkennen, wobei sich der Abstand gemäß den Winkeln der Gelenke ändert, und konfiguriert ist, um den Winkel der Gelenke basierend auf dem erkannten Abstand zu erkennen.

3. Informationsverarbeitungsvorrichtung (100) nach Anspruch 2, wobei
der berührungslose Sensor ein lichtemittierendes Element, das konfiguriert ist, um Licht zu emittieren, und ein lichtempfangendes Element, das konfiguriert ist, um reflektiertes Licht zu erkennen, einschließt,
das lichtemittierende Element konfiguriert ist, um Licht an ein Glied des Fingers zu emittieren,
das lichtempfangende Element konfiguriert ist, um reflektiertes Licht des an das Fingerglied emittierten Lichts zu erkennen, und
die Erkennungseinheit (110) konfiguriert ist, um
den Abstand zu dem Finger basierend auf der Intensität des reflektierten Lichts, das von dem lichtempfangenden Element erkannt wird, zu erkennen.

4. Informationsverarbeitungsvorrichtung (100) nach Anspruch 3, wobei
der berührungslose Sensor konfiguriert ist, um an einem Mittelglied von mindestens einem von einem Zeigefinger, einem Mittelfinger, einem Ringfinger und einem kleinen Finger bereitgestellt zu werden, und
das lichtemittierende Element konfiguriert ist, um Licht an mindestens eines von einem Endglied und einem Grundglied des Fingers zu emittieren,
das lichtempfangende Element konfiguriert ist, um reflektiertes Licht des an mindestens eines von dem Endglied und dem Grundglied des Fingers emittierten Lichts zu erkennen,
die Erkennungseinheit (110) konfiguriert ist, um
einen relativen Winkel des Endglieds des Fingers zum Mittelglied des Fingers basierend auf einem Abstand zwischen dem Endglied des Fingers und dem berührungslosen Sensor, der basierend auf der Intensität des reflektierten Lichts berechnet wird, als einen Winkel eines DIP-Gelenks des Fingers zu berechnen; und
die Schätzungseinheit (152)
konfiguriert ist, um einen relativen Winkel des Grundglieds des Fingers zum Mittelglied des Fingers als einen Winkel eines PIP-Gelenks des Fingers zu berechnen, und konfiguriert ist, um die Haltung des Fingers basierend auf dem Winkel des DIP-Gelenks und dem Winkel des PIP-Gelenks des Fingers zu schätzen.

5. Informationsverarbeitungsvorrichtung (100) nach Anspruch 4, ferner umfassend:
einen ersten Haltungssensor (110A), der konfiguriert ist, um eine Neigung des Mittelglieds des Fingers relativ zu einer Schwerkraftrichtung zu erkennen,
wobei die Schätzungseinheit (152) konfiguriert ist, um
einen Winkel eines MP-Gelenks des Fingers basierend auf dem Winkel des PIP-Gelenks des Fingers und der Neigung des Mittelglieds des Fingers relativ zu der Schwerkraftrichtung, die von dem ersten Haltungssensor (110A) erkannt wird, zu berechnen.

6. Informationsverarbeitungsvorrichtung (100) nach Anspruch 5, ferner umfassend:
einen zweiten Haltungssensor (110C), der konfiguriert ist, um eine Neigung des Rückens einer Hand relativ zu einer Schwerkraftrichtung zu erkennen,
wobei die Schätzungseinheit (152) konfiguriert ist, um
einen relativen Winkel des Grundglieds des Fingers zum Handrücken basierend auf einer Neigung des Handrückens relativ zu der Schwerkraftrichtung, die von dem zweiten Haltungssensor (110C) erkannt wird, als den Winkel des MP-Gelenks zu berechnen.

7. Informationsverarbeitungsvorrichtung (100) nach Anspruch 6, wobei
der zweite Haltungssensor konfiguriert ist, um in einem Bereich zwischen den MP-Gelenken an Grundgliedknochen des Zeigefingers und des Mittelfingers und einem Handgelenk auf dem Handrücken bereitgestellt zu werden; oder wobei
der erste Haltungssensor (110A) konfiguriert ist, um eine Adduktions- oder Abduktionsneigung des Fingers relativ zur Schwerkraftrichtung zu erkennen,
der zweite Haltungssensor (110C) konfiguriert ist, um eine Adduktions- oder Abduktionsneigung einer Handfläche relativ zur Schwerkraftrichtung zu erkennen, und
die Schätzungseinheit (152) konfiguriert ist, um
Informationen über einen relativen Adduktions- oder Abduktionswinkel des Fingers zum Handrücken basierend auf der Adduktions- oder Abduktionsneigung des Fingers relativ zur Schwerkraftrichtung, die von dem ersten Haltungssensor (110A) erkannt wird, und der Adduktions- oder Abduktionsneigung der Handfläche relativ zur Schwerkraftrichtung, die von dem zweiten Haltungssensor (110C) erkannt wird, zu berechnen.

8. Informationsverarbeitungsvorrichtung (100) nach Anspruch 6, ferner umfassend
einen dritten Haltungssensor (110D), der konfiguriert ist, um eine Neigung eines Handgelenks relativ zur Schwerkraftrichtung zu erkennen,
wobei die Schätzungseinheit (152) konfiguriert ist, um
eine Neigung des Handgelenks relativ zur Schwerkraftrichtung in Bezug auf den Handrücken auf Grundlage der von dem dritten Haltungssensor (110D) erkannten Neigung des Handgelenks relativ zur Schwerkraftrichtung und der von dem zweiten Haltungssensor (110C) erkannten Neigung des Handrückens relativ zur Schwerkraftrichtung als einen Neigungswinkel des Handgelenks zu berechnen.

9. Informationsverarbeitungsvorrichtung (100) nach einem der Ansprüche 5 bis 8, wobei
der erste Haltungssensor (110A) ein 9-Achsen-Sensor ist.

10. Informationsverarbeitungsvorrichtung (100) nach einem der Ansprüche 6 bis 8, wobei
der zweite Haltungssensor (110C) ein 9-Achsen-Sensor ist.

11. Informationsverarbeitungsvorrichtung (100) nach Anspruch 8, wobei
der dritte Haltungssensor (110D) ein 9-Achsen-Sensor ist.

12. Informationsverarbeitungsvorrichtung (100) nach einem der Ansprüche 1 bis 11, ferner umfassend:
einen Exoskelettroboter (120), der konfiguriert ist, um die von der Schätzungseinheit geschätzte Haltung des Fingers zu reproduzieren, einschließlich:
eines Remote-Center-of-Motion-Mechanismus, der eine doppelte Parallelogrammstruktur unter Verwendung paralleler Verbindungen aufweist;
wobei ein Rotationsmechanismus konfiguriert ist, um den Remote-Center-of-Motion-Mechanismus gemäß der Adduktions- oder Abduktionsrotation des Fingers zu drehen;
eines Halteelements, das an einem ersten Scheitelpunkt befestigt ist, der diagonal zu einem Verbindungspunkt zweier Parallelogrammstrukturen des Remote-Center-of-Motion-Mechanismus positioniert ist, und konfiguriert ist, um den Finger zu halten;
einer ersten Verbindung (L1), die ein Ende aufweist, das an einem zweiten Scheitelpunkt (P2) befestigt ist, der diagonal zu dem Verbindungspunkt positioniert ist;
einer zweiten Verbindung (L2), die ein Ende aufweist, das durch ein erstes Kugelgelenk (J1) mit einem anderen Ende der ersten Verbindung (L1) verbunden ist; und
einer dritten Verbindung (L3), die ein Ende aufweist, das durch ein zweites Kugelgelenk (J2) mit einem anderen Ende der zweiten Verbindung (L2) verbunden ist;
wobei die erste Verbindung (L1), die zweite Verbindung (L2) und die dritte Verbindung (L3) eine Vierstangenverbindungsstruktur bilden.

13. Informationsverarbeitungsvorrichtung (100) nach Anspruch 12, wobei
der Exoskelettroboter (120) ferner einen Motor (121A) einschließt und die dritte Verbindung (L3) ein anderes Ende aufweist, das an einer Rotationswelle des Motors (121A) befestigt ist und/oder wobei
eine Rotationsachse des Rotationsmechanismus und eine Rotationsachse des Remote-Center-of-Motion-Mechanismus in einer Reihenfolge der Rotationsachse des Rotationsmechanismus und der Rotationsachse des Remote-Center-of-Motion-Mechanismus von nahe einem Handgelenk eines Trägers des Exoskelettroboters angeordnet sind und/oder wobei
eine Rotationsachse des Rotationsmechanismus an einem MP-Gelenk eines Fingers eines Trägers des Exoskelettroboters positioniert ist und eine Rotationsachse des Remote-Center-of-Motion-Mechanismus innerhalb eines vorbestimmten Bereichs vom MP-Gelenk des Fingers positioniert ist, in einer Richtung, in der man vom MP-Gelenk des Fingers des Trägers des Exoskelettroboters auf ein PIP-Gelenk blickt.

14. Informationsverarbeitungsverfahren, umfassend:
Erkennen von Winkeln von mindestens einigen Gelenken eines Fingers auf eine berührungslose Weise durch eine Erkennungseinheit (110) und
Schätzen einer Haltung des Fingers basierend auf den erkannten Winkeln der Gelenke.

15. Computerprogramm, umfassend Anweisungen, die die Informationsverarbeitungsvorrichtung nach Anspruch 1 veranlassen, das Informationsverarbeitungsverfahren nach Anspruch 14 auszuführen.

## Revendications

1. Dispositif de traitement d'informations (100) comprenant :
une unité de détection (110) configurée pour détecter sans contact des angles d'au moins certaines articulations d'un doigt ; et
une unité d'estimation (152) configurée pour estimer une posture du doigt sur la base des angles des articulations détectés par l'unité de détection.

2. Dispositif de traitement d'informations (100) selon la revendication 1, dans lequel
l'unité de détection (110) comporte un capteur sans contact, est configurée pour détecter une distance entre le capteur sans contact et le doigt, la distance variant en fonction des angles des articulations, et est configurée pour détecter les angles des articulations sur la base de la distance détectée.

3. Dispositif de traitement d'informations (100) selon la revendication 2, dans lequel
le capteur sans contact comporte un élément électroluminescent configuré pour émettre une lumière et un élément de réception de lumière configuré pour détecter une lumière réfléchie de la lumière,
l'élément électroluminescent est configuré pour émettre une lumière vers une phalange du doigt,
l'élément de réception de lumière est configuré pour détecter une lumière réfléchie de la lumière émise vers la phalange du doigt, et
l'unité de détection (110) est configurée pour
détecter la distance par rapport au doigt sur la base d'une intensité de la lumière réfléchie détectée par l'élément de réception de lumière.

4. Dispositif de traitement d'informations (100) selon la revendication 3, dans lequel
le capteur sans contact est configuré pour être prévu au niveau d'une phalange moyenne d'au moins l'un parmi un index, un majeur, un annulaire, et un auriculaire, et
l'élément électroluminescent est configuré pour émettre une lumière vers au moins l'une parmi une phalange distale et une phalange proximale du doigt,
l'élément de réception de lumière est configuré pour détecter une lumière réfléchie de la lumière émise vers au moins l'une parmi la phalange distale et la phalange proximale du doigt,
l'unité de détection (110) est configurée pour
calculer, en tant qu'angle d'une articulation DIP du doigt, un angle relatif entre la phalange distale du doigt et la phalange moyenne du doigt, sur la base d'une distance entre la phalange distale du doigt et le capteur sans contact, calculée sur la base de l'intensité de la lumière réfléchie ; et
l'unité d'estimation (152)
est configurée pour calculer, en tant qu'angle d'une articulation PIP du doigt, un angle relatif entre la phalange proximale du doigt et la phalange moyenne du doigt, et configurée pour estimer la posture du doigt sur la base de l'angle de l'articulation DIP et l'angle de l'articulation PIP du doigt.

5. Dispositif de traitement d'informations (100) selon la revendication 4, comprenant en outre
un premier capteur de posture (110A) configuré pour détecter une inclinaison de la phalange moyenne du doigt par rapport à une direction de gravité,
dans lequel l'unité d'estimation (152) est configurée pour
calculer un angle d'une articulation MP du doigt, sur la base de l'angle de l'articulation PIP du doigt et de l'inclinaison de la phalange moyenne du doigt par rapport à la direction de gravité détectée par le premier capteur de posture (110A).

6. Dispositif de traitement d'informations (100) selon la revendication 5, comprenant en outre
un deuxième capteur de posture (110C) configuré pour détecter une inclinaison du dos d'une main par rapport à une direction de gravité,
dans lequel l'unité d'estimation (152) est configurée pour
calculer, en tant qu'angle de l'articulation MP, un angle relatif entre la phalange proximale du doigt et le dos de la main, sur la base d'une inclinaison du dos de la main par rapport à la direction de gravité détectée par le deuxième capteur de posture (110C).

7. Dispositif de traitement d'informations (100) selon la revendication 6, dans lequel
le deuxième capteur de posture est configuré pour être prévu dans une zone entre les articulations MP sur des os de phalange proximale de l'index et du majeur et un poignet sur le dos de la main ; ou dans lequel
le premier capteur de posture (110A) est configuré pour détecter une inclinaison d'adduction ou d'abduction du doigt par rapport à la direction de gravité,
le deuxième capteur de posture (110C) est configuré pour détecter une inclinaison d'adduction ou d'abduction d'une paume par rapport à la direction de gravité, et
l'unité d'estimation (152) est configurée pour
calculer des informations sur un angle relatif d'adduction ou d'abduction du doigt par rapport au dos de la main sur la base de l'inclinaison d'adduction ou d'abduction du doigt par rapport à la direction de gravité détectée par le premier capteur de posture (110A) et de l'inclinaison d'adduction ou d'abduction de la paume par rapport à la direction de gravité détectée par le deuxième capteur de posture (110C).

8. Dispositif de traitement d'informations (100) selon la revendication 6, comprenant en outre
un troisième capteur de posture (110D) configuré pour détecter une inclinaison d'un poignet par rapport à la direction de gravité,
dans lequel l'unité d'estimation (152) est configurée pour
calculer, en tant qu'angle d'inclinaison du poignet, une inclinaison du poignet par rapport à la direction de gravité par rapport au dos de la main, sur la base de l'inclinaison du poignet par rapport à la direction de gravité détectée par le troisième capteur de posture (110D) et de l'inclinaison du dos de la main par rapport à la direction de gravité détectée par le deuxième capteur de posture (110C).

9. Dispositif de traitement d'informations (100) selon l'une quelconque des revendications 5 à 8, dans lequel
le premier capteur de posture (110A) est un capteur à 9 axes.

10. Dispositif de traitement d'informations (100) selon l'une quelconque des revendications 6 à 8, dans lequel
le deuxième capteur de posture (110C) est un capteur à 9 axes.

11. Dispositif de traitement d'informations (100) selon la revendication 8, dans lequel
le troisième capteur de posture (110D) est un capteur à 9 axes.

12. Dispositif de traitement d'informations (100) selon l'une quelconque des revendications 1 à 11, comprenant en outre :
un robot exosquelette (120) configuré pour reproduire la posture du doigt estimée par l'unité d'estimation comportant :
un mécanisme de centre de mouvement distant qui a une structure en double parallélogramme utilisant des biellettes parallèles ;
un mécanisme de rotation est configuré pour faire tourner le mécanisme de centre de mouvement distant en fonction d'une rotation d'adduction ou d'abduction du doigt ;
un élément de retenu qui est fixé à un premier vertex positionné en diagonale par rapport à un point de liaison de deux structures en parallélogramme du mécanisme de centre de mouvement distant et configuré pour retenir le doigt ;
une première biellette (L1) ayant une extrémité fixée à un second vertex (P2) positionné en diagonale par rapport au point de liaison ;
une deuxième biellette (L2) ayant une extrémité reliée à une autre extrémité de la première biellette (L1) par un premier joint à rotule (J1) ; et
une troisième biellette (L3) ayant une extrémité reliée à une autre extrémité de la deuxième biellette (L2) par un second joint à rotule (J2) ;
dans lequel la première biellette (L1), la deuxième biellette (L2) et la troisième biellette (L3) constituent une structure à quatre barres.

13. Dispositif de traitement d'informations (100) selon la revendication 12, dans lequel
le robot exosquelette (120) comporte en outre un moteur (121A), et la troisième biellette (L3) a une autre extrémité qui est fixée à un arbre de rotation du moteur (121A) ; et/ou dans lequel
un axe de rotation du mécanisme de rotation et un axe de rotation du mécanisme de centre de mouvement distant sont disposés dans l'ordre de l'axe de rotation du mécanisme de rotation et de l'axe de rotation du mécanisme de centre de mouvement distant, à partir d'un poignet d'un porteur du robot exosquelette ; et/ou dans lequel
un axe de rotation du mécanisme de rotation est positionné au niveau d'une articulation MP d'un doigt d'un porteur du robot exosquelette, et un axe de rotation du mécanisme de centre de mouvement distant est positionné à l'intérieur d'une plage prédéterminée à partir de l'articulation MP du doigt, dans une direction d'observation d'une articulation PIP à partir de l'articulation MP du doigt du porteur du robot exosquelette.

14. Procédé de traitement d'informations comprenant :
la détection sans contact, par une unité de détection (110), d'angles d'au moins certaines articulations d'un doigt ; et
l'estimation d'une posture du doigt sur la base des angles détectés des articulations.

15. Programme informatique comprenant des instructions destinées à amener le dispositif de traitement d'informations selon la revendication 1 à exécuter le procédé de traitement d'informations selon la revendication 14.
